# FASCICULE DE BREVET EUROPEEN

(11) **EP 1 519 960 B1**
(45) Date de publication et mention de la délivrance du brevet: **11.11.2009**
(21) Numéro de dépôt: 03755580.2
(22) Date de dépôt: 07.07.2003
(51) Int. Cl.: C07K 16/28, A61K 39/395, A61P 17/06, A61P 31/18, A61P 35/00, A61P 37/06

(54) **FRAGMENTS FAB MUTANS DE L'ANTICORPS ANTI-CD4 CHIMERE 13B8.2 ET LEURS APPLICATIONS**
MUTANTE FAB FRAGMENTE DES CHIMÄREN ANTI-CD4 ANTIKÖRPERS 13B8.2 UND IHRE VERWENDUNGEN
MUTANT FAB FRAGMENTS OF THE CHIMERIC 13B8.2 ANTI-CD4 ANTIBODY AND USES THEREOF

(30) Priorité: 05.07.2002 FR 0208486
(43) Date de publication de la demande: 06.04.2005
(73) Titulaire: CENTRE NATIONAL DE LA RECHERCHE SCIENTIFIQUE (CNRS), 75794 Paris Cedex 16 (FR)
(72) Inventeur: DEVAUX, Christian, 361, rue du Pré aux Clercs, F-34090 Montpellier (FR); BES, Cédric, F-34090 Montpellier (FR); BRIANT-LONGUET, Laurence, F-30660 Gallargues (FR); CERUTTI, Martine, F-30380 Saint-Christol-les-Alès (FR); DEVAUCHELLE, Gérard, F-30380 Saint-Christol-les-Alès (FR); CHARDES, Thierry, F-34820 Assas (FR); GRANIER, Claude, F-34830 Clapiers (FR); PAU, Bernard, F-34080 Montpellier (FR)
(74) Mandataire: Breese, Pierre
(86) Numéro de dépôt international: PCT/FR2003/002108
(87) Numéro de publication internationale: WO 2004/005350

(56) Documents cités:
- BES CEDRIC ET AL: "The chimeric mouse-human anti-CD4 Fab 13B8.2 expressed in baculovirus inhibits both antigen presentation and HIV-1 promoter activation." HUMAN ANTIBODIES, vol. 10, no. 2, 2001, pages 67-76, XP008015891 ISSN: 1093-2607 cité dans la demande
- BES^A C ET AL: "Efficient CD4 binding and immunosuppressive properties of the 13B8.2 monoclonal antibody are displayed by its CDR-H1-derived peptide CB1" FEBS LETTERS, ELSEVIER SCIENCE PUBLISHERS, AMSTERDAM, NL, vol. 508, no. 1, 9 novembre 2001 (2001-11-09), pages 67-74, XP004322279 ISSN: 0014-5793 cité dans la demande
- COUDRONNIERE NOLWENN ET AL: "A novel complex of proteins binds the HIV-1 promoter upon virus interaction with CD4." JOURNAL OF BIOMEDICAL SCIENCE, vol. 5, no. 4, juillet 1998 (1998-07), pages 281-289, XP008015890 ISSN: 1021-7770
- BÈS CÉDRIC ET AL: "Mapping the paratope of anti-CD4 recombinant Fab 13B8.2 by combining parallel peptide synthesis and site-directed mutagenesis." THE JOURNAL OF BIOLOGICAL CHEMISTRY. UNITED STATES 18 APR 2003, vol. 278, no. 16, 18 avril 2003 (2003-04-18), pages 14265-14273, XP004434091 ISSN: 0021-9258
- CASSET FLORENCE ET AL: "A peptide mimetic of an anti-CD4 monoclonal antibody by rational design." BIOCHEMICAL AND BIOPHYSICAL RESEARCH COMMUNICATIONS. UNITED STATES 18 JUL 2003, vol. 307, no. 1, 18 juillet 2003 (2003-07-18), pages 198-205, XP002264147 ISSN: 0006-291X

### LISTAGE DE SEQUENCES

<110> CENTRE NATIONAL DE LA RECHERCHE SCIENTIFIQUE (CNRS)
<120> FRAGMENTS Fab MUTANTS DE L'ANTICORPS ANTI-CD4 CHIMERE 13B8.2 ET LEURS APPLICATIONS.
<130> 27324-CNRS-DEVAUX Fab-CD4
<140> PCT/FR03/XXXXX
   <141> 2003-07-07
<150> FR02/08486
   <151> 2002-07-05
<160> 58
<170> PatentIn version 3.1
<210> 1
   <211> 117
   <212> PRT
   <213> mus musculus
   <220>
   <221> MISC_FEATURE
   <222> (1)..(117)
   <223> Région VH2-DQ52-JH3 du domaine VH de l'anticorps 13B8.2
   <300>
   <301> Bes,C., Briant-Longuet,L., Cerruti,M., De Berardinis,P., Devauchelle,G., Devaux,C., Granier,C. and Chardes,T
   <302> Efficient CD4 binding and immunosuppressive properties of the 13B8.2 monoclonal antibody are displayed by its CDR-H1-derived peptide CB1(1).
   <303> FEBS Letters
   <304> 508
   <305> 1
   <306> 67-74
   <307> 2001
   <308> GENEBANK/CAC08525
   <309> 2001-12-13
   <313> (1)..(117)
   <400> 1
   Gln Val Gln Leu Lys Gln Ser Gly Pro Gly Leu Val Gln Pro Ser Gln
   1               5                   10                  15
   Ser Leu Ser Ile Thr Cys Thr Val Ser Gly Phe Ser Leu Thr Thr Phe
               20                  25                  30
   Gly Val His Trp Val Arg Gln Ser Pro Gly Lys Gly Leu Glu Trp Leu
           35                  40                  45
   Gly Val Ile Trp Arg Ser Gly Ile Thr Asp Tyr Asn Val Pro Phe Met
       50                  55                  60
   Ser Arg Leu Ser Ile Thr Lys Asp Asn Ser Lys Ser Gln Val Phe Phe
   65                  70                  75                  80
   Lys Leu Asn Ser Leu Gln Pro Asp Asp Thr Ala Ile Tyr Tyr Cys Ala
                   85                  90                  95
   Lys Asn Asp Pro Gly Thr Gly Phe Ala Tyr Trp Gly Gln Gly Thr Leu
               100                 105                 110
   Val Thr Val Ser Ala
           115
<210> 2
   <211> 107
   <212> PRT
   <213> mus musculus
<220>
   <221> MISC_FEATURE
   <222> (1)..(107)
   <223> Région VK12/13-Jk1 de la chaîne kappa de l'anticorps 13B8.2
   <300>
   <301> Bes,C., Briant-Longuet,L., Cerruti,M., De Berardinis,P; Devauchelle,G., Devaux,C., Granier,C. and Chardes,T.
   <302> Efficient CD4 binding and immunosuppressive properties of the 13B8.2 monoclonal antibody are displayed by its CDR-H1-derived peptide CB1(1).
   <303> FEBS Letters
   <304> 508
   <305> 1
   <306> 67-74
   <307> 2001
   <308> GENBANK/CAC08524
   <309> 2001-12-13
   <313> (1)..(107)
   <400> 2
   Asp Ile Gln Met Thr Gln Ser Pro Ala Ser Leu Ser Ala Ser Val Gly
   1               5                   10                  15
   Glu Thr Val Thr Phe Thr Cys Arg Ala Ser Glu Asn Ile Tyr Ser Tyr
               20                  25                  30
   Leu Ala Trp Tyr Gln Gln Lys Gln Gly Lys Ser Pro Gln Leu Leu Val
           35                  40                  45
   His Asp Ala Lys Thr Leu Ala Glu Gly Val Pro Ser Arg Phe Ser Gly
       50                  55                  60
   Gly Gly Ser Gly Thr Gln Phe Ser Leu Lys Ile Asn Thr Leu Gln Pro
   65                  70                  75                  80
   Glu Asp Phe Gly Thr Tyr Tyr Cys Gln His His Tyr Gly Asn Pro Pro
                   85                  90                  95
   Thr Phe Gly Gly Gly Thr Lys Leu Glu Ile Lys
               100                 105
<210> 3
   <211> 117
   <212> PRT
   <213> Artificial
   <220>
   <221> MISC_FEATURE
   <222> (1)..(117)
   <223> Région mutée du Fab recombinant mutant FH32
   <220>
   <221> MISC_FEATURE
   <222> (32)..(32)
   <223> F muté par A
   <400> 3
   Gln Val Gln Leu Lys Gln Ser Gly Pro Gly Leu Val Gln Pro Ser Gln
   1               5                   10                  15
   Ser Leu Ser Ile Thr Cys Thr Val Ser Gly Phe Ser Leu Thr Thr Ala
               20                  25                  30
   Gly Val His Trp Val Arg Gln Ser Pro Gly Lys Gly Leu Glu Trp Leu
           35                  40                  45
   Gly Val Ile Trp Arg Ser Gly Ile Thr Asp Tyr Asn Val Pro Phe Met
       50                  55                  60
   Ser Arg Leu Ser Ile Thr Lys Asp Asn Ser Lys Ser Gln Val Phe Phe
   65                  70                  75                  80
   Lys Leu Asn Ser Leu Gln Pro Asp Asp Thr Ala Ile Tyr Tyr Cys Ala
                   85                  90                  95
   Lys Asn Asp Pro Gly Thr Gly Phe Ala Tyr Trp Gly Gln Gly Thr Leu
               100                 105                 110
   Val Thr Val Ser Ala
           115
<210> 4
   <211> 117
   <212> PRT<213> Artificial
   <220>
   <221> MISC_FEATURE
   <222> (1)..(117)
   <223> Région mutée du Fab recombinant mutant H35-H
   <220>
   <221> MISC_FEATURE
   <222> (35) .. (35)
   <223> H muté par A
<400> 4
   Gln Val Gln Leu Lys Gln Ser Gly Pro Gly Leu Val Gln Pro Ser Gln
   1               5                   10                  15
   Ser Leu Ser Ile Thr Cys Thr Val Ser Gly Phe Ser Leu Thr Thr Phe
               20                  25                  30
   Gly Val Ala Trp Val Arg Gln Ser Pro Gly Lys Gly Leu Glu Trp Leu
           35                  40                  45
   Gly Val Ile Trp Arg Ser Gly Ile Thr Asp Tyr Asn Val Pro Phe Met
       50                  55                  60
   Ser Arg Leu Ser Ile Thr Lys Asp Asn Ser Lys Ser Gln Val Phe Phe
   65                  70                  75                  80
   Lys Leu Asn Ser Leu Gln Pro Asp Asp Thr Ala Ile Tyr Tyr Cys Ala
                   85                  90                  95
   Lys Asn Asp Pro Gly Thr Gly Phe Ala Tyr Trp Gly Gln Gly Thr Leu
               100                 105                 110
   Val Thr Val Ser Ala
           115
<210> 5
   <211> 117
   <212> PRT
   <213> Artificial
   <220>
   <221> MISC_FEATURE
   <222> (1)..(117)
   <223> Région mutée du Fab recombinant mutant W36-H
   <220>
   <221> MISC_FEATURE
   <222> (36) .. (36)
   <223> W muté par A
<400> 5
   Gln Val Gln Leu Lys Gln Ser Gly Pro Gly Leu Val Gln Pro Ser Gln
   1               5                   10                  15
   Ser Leu Ser Ile Thr Cys Thr Val Ser Gly Phe Ser Leu Thr Thr Phe
               20                  25                  30
   Gly Val His Ala Val Arg Gln Ser Pro Gly Lys Gly Leu Glu Trp Leu
           35                  40                  45
   Gly Val Ile Trp Arg Ser Gly Ile Thr Asp Tyr Asn Val Pro Phe Met
       50                  55                  60
   Ser Arg Leu Ser Ile Thr Lys Asp Asn Ser Lys Ser Gln Val Phe Phe
   65                  70                  75                  80
   Lys Leu Asn Ser Leu Gln Pro Asp Asp Thr Ala Ile Tyr Tyr Cys Ala
                   85                  90                  95
   Lys Asn Asp Pro Gly Thr Gly Phe Ala Tyr Trp Gly Gln Gly Thr Leu
               100                 105                 110
   Val Thr Val Ser Ala
           115
<210> 6
   <211> 117
   <212> PRT
   <213> Artificial
   <220>
   <221> MISC_FEATURE
   <222> (1) .. (117)
   <223> Région mutée du Fab recombinant mutant R38-H
   <220>
   <221> MISC_FEATURE
   <222> (38) .. (38)
   <223> H muté par A
<400> 6
   Gln Val Gln Leu Lys Gln Ser Gly Pro Gly Leu Val Gln Pro Ser Gln
   1               5                   10                  15
   Ser Leu Ser Ile Thr Cys Thr Val Ser Gly Phe Ser Leu Thr Thr Phe
               20                  25                  30
   Gly Val His Trp Val Ala Gln Ser Pro Gly Lys Gly Leu Glu Trp Leu
           35                  40                  45
   Gly Val Ile Trp Arg Ser Gly Ile Thr Asp Tyr Asn Val Pro Phe Met
       50                  55                  60
   Ser Arg Leu Ser Ile Thr Lys Asp Asn Ser Lys Ser Gln Val Phe Phe
   65                  70                  75                  80
   Lys Leu Asn Ser Leu Gln Pro Asp Asp Thr Ala Ile Tyr Tyr Cys Ala
                   85                  90                  95
   Lys Asn Asp Pro Gly Thr Gly Phe Ala Tyr Trp Gly Gln Gly Thr Leu
               100                 105                 110
   Val Thr Val Ser Ala
           115
<210> 7
   <211> 117
   <212> PRT
   <213> Artificial
   <220>
   <221> MISC_FEATURE
   <222> (1)..(117)
   <223> Région mutée du Fab recombinant mutant W52-H
   <220>
   <221> MISC_FEATURE
   <222> (52)..(52)
   <223> W muté par A
   <400> 7
   Gln Val Gln Leu Lys Gln Ser Gly Pro Gly Leu Val Gln Pro Ser Gln
   1               5                   10                  15
   Ser Leu Ser Ile Thr Cys Thr Val Ser Gly Phe Ser Leu Thr Thr Phe
   20 25 30
   Gly Val His Trp Val Arg Gln Ser Pro Gly Lys Gly Leu Glu Trp Leu
   35 40 45
   Gly Val Ile Ala Arg Ser Gly Ile Thr Asp Tyr Asn Val Pro Phe Met
   50 55 60
   Ser Arg Leu Ser Ile Thr Lys Asp Asn Ser Lys Ser Gln Val Phe Phe
   65 70 75 80
   Lys Leu Asn Ser Leu Gln Pro Asp Asp Thr Ala Ile Tyr Tyr Cys Ala
   85 90 95
   Lys Asn Asp Pro Gly Thr Gly Phe Ala Tyr Trp Gly Gln Gly Thr Leu
   100 105 110
   Val Thr Val Ser Ala
   115
<210> 8
   <211> 117
   <212> PRT
   <213> Artificial
   <220>
   <221> MISC_FEATURE
   <222> (1)..(117)
   <223> Région mutée du Fab recombinant mutant R53-H
   <220>
   <221> MISC_FEATURE
   <222> (53)..(53)
   <223> R muté par A
   <400> 8
   Gln Val Gln Leu Lys Gln Ser Gly Pro Gly Leu Val Gln Pro Ser Gln
   1 5 10 15
   Ser Leu Ser Ile Thr Cys Thr Val Ser Gly Phe Ser Leu Thr Thr Phe
   20 25 30
   Gly Val His Trp Val Arg Gln Ser Pro Gly Lys Gly Leu Glu Trp Leu
   35 40 45
   Gly Val Ile Trp Ala Ser Gly Ile Thr Asp Tyr Asn Val Pro Phe Met
   50 55 60
   Ser Arg Leu Ser Ile Thr Lys Asp Asn Ser Lys Ser Gln Val Phe Phe
   65 70 75 80
   Lys Leu Asn Ser Leu Gln Pro Asp Asp Thr Ala Ile Tyr Tyr Cys Ala
   85 90 95
   Lys Asn Asp Pro Gly Thr Gly Phe Ala Tyr Trp Gly Gln Gly Thr Leu
   100 105 110
   Val Thr Val Ser Ala
   115
<210> 9
   <211> 117
   <212> PRT
   <213> Artificial
   <220>
   <221> MISC_FEATURE
   <222> (1)..(117)
   <223> Région mutée du Fab recombinant mutant C92-H
   <220>
   <221> MISC_FEATURE
   <222> (92) .. (92)
   <223> C muté par A
<400>
<210> 10
   <211> 117
   <212> PRT
   <213> Artificial
   <220>
   <221> MISC_FEATURE
   <222> (1)..(117)
   <223> Région mutée du Fab recombinant mutant F100K-H
   <220>
   <221> MISC_FEATURE
   <223> Région mutée du Fab recombinant mutant F100K-H Résidu H , en position 100K , muté par A
   <400> 10
   Gln Val Gln Leu Lys Gln Ser Gly Pro Gly Leu Val Gln Pro Ser Gln
   1               5                   10                  15
   Ser Leu Ser Ile Thr Cys Thr Val Ser Gly Phe Ser Leu Thr Thr Phe
               20                  25                  30
   Gly Val His Trp Val Arg Gln Ser Pro Gly Lys Gly Leu Glu Trp Leu
           35                  40                  45
   Gly Val Ile Trp Arg Ser Gly Ile Thr Asp Tyr Asn Val Pro Phe Met
       50                  55                  60
   Ser Arg Leu Ser Ile Thr Lys Asp Asn Ser Lys Ser Gln Val Phe Phe
   65                  70                  75                  80
   Lys Leu Asn Ser Leu Gln Pro Asp Asp Thr Ala Ile Tyr Tyr Cys Ala
                   85                  90                  95
   Lys Asn Asp Pro Gly Thr Gly Ala Ala Tyr Trp Gly Gln Gly Thr Leu
               100                 105                 110
   Val Thr Val Ser Ala
           115
<210> 11
   <211> 117
   <212> PRT
   <213> Artificial
   <220>
   <221> MISC_FEATURE
   <222> (1)..(117)
   <223> Région mutée du Fab recombinant mutant Y102-H
   <220>
   <221> MISC_FEATURE
   <222> (102)..(102)
   <223> Y muté par A
   <400> 11
   Gln Val Gln Leu Lys Gln Ser Gly Pro Gly Leu Val Gln Pro Ser Gln
   1               5                   10                  15
   Ser Leu Ser Ile Thr Cys Thr Val Ser Gly Phe Ser Leu Thr Thr Phe
               20                  25                  30
   Gly Val His Trp Val Arg Gln Ser Pro Gly Lys Gly Leu Glu Trp Leu
           35                  40                  45
   Gly Val Ile Trp Arg Ser Gly Ile Thr Asp Tyr Asn Val Pro Phe Met
       50                  55                  60
   Ser Arg Leu Ser Ile Thr Lys Asp Asn Ser Lys Ser Gln Val Phe Phe
   65                  70                  75                  80
   Lys Leu Asn Ser Leu Gln Pro Asp Asp Thr Ala Ile Tyr Tyr Cys Ala
                   85                  90                  95
   Lys Asn Asp Pro Gly Thr Gly Phe Ala Ala Trp Gly Gln Gly Thr Leu
               100                 105                 110
   Val Thr Val Ser Ala
           115
<210> 12
   <211> 117
   <212> PRT
   <213> Artificial
   <220>
   <221> MISC_FEATURE
   <222> (1)..(117)
   <223> Région mutée du Fab recombinant mutant W103-H
   <220>
   <221> MISC_FEATURE
   <222> (103)..(103)
   <223> W muté par A
   <400> 12
   Gln Val Gln Leu Lys Gln Ser Gly Pro Gly Leu Val Gln Pro Ser Gln
   1               5                   10                  15
   Ser Leu Ser Ile Thr Cys Thr Val Ser Gly Phe Ser Leu Thr Thr Phe
               20                  25                  30
   Gly Val His Trp Val Arg Gln Ser Pro Gly Lys Gly Leu Glu Trp Leu
           35                  40                  45
   Gly Val Ile Trp Arg Ser Gly Ile Thr Asp Tyr Asn Val Pro Phe Met
       50                  55                  60
   Ser Arg Leu Ser Ile Thr Lys Asp Asn Ser Lys Ser Gln Val Phe Phe
   65                  70                  75                  80
   Lys Leu Asn Ser Leu Gln Pro Asp Asp Thr Ala Ile Tyr Tyr Cys Ala
                   85                  90                  95
   Lys Asn Asp Pro Gly Thr Gly Phe Ala Tyr Ala Gly Gln Gly Thr Leu
               100                 105                 110
   Val Thr Val Ser Ala
           115
<210> 13
   <211> 107
   <212> PRT
   <213> Artificial
   <220>
   <221> MISC_FEATURE
   <222> (1)..(107)
   <223> Région VL mutée du Fab recombinant mutant Y32-L
   <220>
   <221> MISC_FEATURE
   <222> (32)..(32)
   <223> Y muté par A
   <400> 13
   Asp Ile Gln Met Thr Gln Ser Pro Ala Ser Leu Ser Ala Ser Val Gly
   1 5 10 15
   Glu Thr Val Thr Phe Thr Cys Arg Ala Ser Glu Asn Ile Tyr Ser Ala
   20 25 30
   Leu Ala Trp Tyr Gln Gln Lys Gln Gly Lys Ser Pro Gln Leu Leu Val
   35 40 45
   His Asp Ala Lys Thr Leu Ala Glu Gly Val Pro Ser Arg Phe Ser Gly
   50 55 60
   Gly Gly Ser Gly Thr Gln Phe Ser Leu Lys Ile Asn Thr Leu Gln Pro
   65 70 75 80
   Glu Asp Phe Gly Thr Tyr Tyr Cys Gln His His Tyr Gly Asn Pro Pro
   85 90 95
   Thr Phe Gly Gly Gly Thr Lys Leu Glu Ile Lys
   100 105
<210> 14
   <211> 107
   <212> PRT
   <213> Artificial
   <220>
   <221> MISC_FEATURE
   <222> (1)..(107)
   <223> Région VL mutée du Fab recombinant mutant W35-L
   <220>
   <221> MISC_FEATURE
   <222> (35)..(35)
   <223> W muté par A
   <400> 14
   Asp Ile Gln Met Thr Gln Ser Pro Ala Ser Leu Ser Ala Ser Val Gly
   1 5 10 15
   Glu Thr Val Thr Phe Thr Cys Arg Ala Ser Glu Asn Ile Tyr Ser Tyr
   20 25 30
   Leu Ala Ala Tyr Gln Gln Lys Gln Gly Lys Ser Pro Gln Leu Leu Val
   35 40 45
   His Asp Ala Lys Thr Leu Ala Glu Gly Val Pro Ser Arg Phe Ser Gly
   50 55 60
   Gly Gly Ser Gly Thr Gln Phe Ser Leu Lys Ile Asn Thr Leu Gln Pro
   65 70 75 80
   Glu Asp Phe Gly Thr Tyr Tyr Cys Gln His His Tyr Gly Asn Pro Pro
   85 90 95
   Thr Phe Gly Gly Gly Thr Lys Leu Glu Ile Lys
   100 105
<210> 15
   <211> 107
   <212> PRT
   <213> Artificial
   <220>
   <221> MISC_FEATURE
   <222> (1)..(107)
   <223> Région VL mutée du Fab recombinant mutant Y36-L
   <220>
   <221> MISC_FEATURE
   <222> (36)..(36)
   <223> Y muté par A
   <400> 15
   Asp Ile Gln Met Thr Gln Ser Pro Ala Ser Leu Ser Ala Ser Val Gly
   1               5                   10                  15
   Glu Thr Val Thr Phe Thr Cys Arg Ala Ser Glu Asn Ile Tyr Ser Tyr
               20                  25                  30
   Leu Ala Trp Ala Gln Gln Lys Gln Gly Lys Ser Pro Gln Leu Leu Val
           35                  40                  45
   His Asp Ala Lys Thr Leu Ala Glu Gly Val Pro Ser Arg Phe Ser Gly
       50                  55                  60
   Gly Gly Ser Gly Thr Gln Phe Ser Leu Lys Ile Asn Thr Leu Gln Pro
   65                  70                  75                  80
   Glu Asp Phe Gly Thr Tyr Tyr Cys Gln His His Tyr Gly Asn Pro Pro
                   85                  90                  95
   Thr Phe Gly Gly Gly Thr Lys Leu Glu Ile Lys
               100                 105
<210> 16
   <211> 107
   <212> PRT
   <213> Artificial
   <220>
   <221> MISC_FEATURE
   <222> (1)..(107)
   <223> Région mutée du Fab recombinant mutant C88-L
   <220>
   <221> MISC_FEATURE
   <222> (88)..(88)
   <223> C muté par A
   <400> 16
   Asp Ile Gln Met Thr Gln Ser Pro Ala Ser Leu Ser Ala Ser Val Gly
   1 5 10 15
   Glu Thr Val Thr Phe Thr Cys Arg Ala Ser Glu Asn Ile Tyr Ser Tyr
   20 25 30
   Leu Ala Trp Tyr Gln Gln Lys Gln Gly Lys Ser Pro Gln Leu Leu Val
   35 40 45
   His Asp Ala Lys Thr Leu Ala Glu Gly Val Pro Ser Arg Phe Ser Gly
   50 55 60
   Gly Gly Ser Gly Thr Gln Phe Ser Leu Lys Ile Asn Thr Leu Gln Pro
   65 70 75 80
   Glu Asp Phe Gly Thr Tyr Tyr Ala Gln His His Tyr Gly Asn Pro Pro
   85 90 95
   Thr Phe Gly Gly Gly Thr Lys Leu Glu Ile Lys
   100 105
<210> 17
   <211> 107
   <212> PRT
   <213> Artificial
   <220>
   <221> MISC_FEATURE
   <222> (1)..(107)
   <223> Région mutée du Fab recombinant mutant H91-L
   <220>
   <221> MISC_FEATURE
   <222> (91)..(91)
   <223> H muté par A
   <400> 17
   Asp Ile Gln Met Thr Gln Ser Pro Ala Ser Leu Ser Ala Ser Val Gly
   1 5 10 15
   Glu Thr Val Thr Phe Thr Cys Arg Ala Ser Glu Asn Ile Tyr Ser Tyr
   20 25 30
   Leu Ala Trp Tyr Gln Gln Lys Gln Gly Lys Ser Pro Gln Leu Leu Val
   35 40 45
   His Asp Ala Lys Thr Leu Ala Glu Gly Val Pro Ser Arg Phe Ser Gly
   50 55 60
   Gly Gly Ser Gly Thr Gln Phe Ser Leu Lys Ile Asn Thr Leu Gln Pro
   65 70 75 80
   Glu Asp Phe Gly Thr Tyr Tyr Cys Gln His Ala Tyr Gly Asn Pro Pro
   85 90 95
   Thr Phe Gly Gly Gly Thr Lys Leu Glu Ile Lys
   100 105
<210> 18
   <211> 107
   <212> PRT
   <213> Artificial
   <220>
   <221> MISC_FEATURE
   <222> (1)..(107)
   <223> Région mutée du Fab recombinant mutant Y92-L
   <220>
   <221> MISC_FEATURE
   <222> (92)..(92)
   <223> Y muté par A
   <400> 18
   Asp Ile Gln Met Thr Gln Ser Pro Ala Ser Leu Ser Ala Ser Val Gly
   1               5                   10                  15
   Glu Thr Val Thr Phe Thr Cys Arg Ala Ser Glu Asn Ile Tyr Ser Tyr
               20                  25                  30
   Leu Ala Trp Tyr Gln Gln Lys Gln Gly Lys Ser Pro Gln Leu Leu Val
           35                  40                  45
   His Asp Ala Lys Thr Leu Ala Glu Gly Val Pro Ser Arg Phe Ser Gly
       50                  55                  60
   Gly Gly Ser Gly Thr Gln Phe Ser Leu Lys Ile Asn Thr Leu Gln Pro
   65                  70                  75                  80
   Glu Asp Phe Gly Thr Tyr Tyr Cys Gln His His Ala Gly Asn Pro Pro
                   85                  90                  95
   Thr Phe Gly Gly Gly Thr Lys Leu Glu Ile Lys
               100                 105
<210> 19
   <211> 20
   <212> DNA
   <213> Artificial
   <220>
   <221> amorce
   <222> (1)..(20)
   <223> Amorce directe pour l'amplification du domaine variable de la chaîne lourde de l'anticorps 13B8.2
   <400> 19
   atccggaaca atgtcgccgg 20
<210> 20
   <211> 20
   <212> DNA
   <213> Artificial
   <220>
   <221> amorce
   <222> (1)..(20)
   <223> Amorce indirecte pour l'amplification du domaine variable de la chaîne lourde de l'anticorps 13B8.2
   <400> 20
   catcacttac aacaaggggg 20
<210> 21
   <211> 18
   <212> DNA
   <213> Artificial
   <220>
   <221> amorce
   <222> (1)..(18)
   <223> Amorce directe pour l'amplification du domaine variable de la chaîne légère de l'anticorps 13B8.2
   <400> 21
   tatcagcccc agcgttgc 18
<210> 22
   <211> 18
   <212> DNA
   <213> Artificial <220><221> amorce <222> (1)..(18)
   <223> Amorce indirecte pour l'amplification du domaine variable de la chaîne lourde de l'anticorps 13B8.2
   <400> 22
   ctgcgagcag ttgtttgt 18
<210> 23
   <211> 21
   <212> DNA
   <213> Artificial
   <220>
   <221> amorce
   <222> (1)..(21)
   <223> Amorce directe pour l'amplification du domaine variable de la chaîne lourde du Fab mutant F32-H.
   <400> 23
   actaccgctg gtgtacactg g 21
<210> 24
   <211> 20
   <212> DNA
   <213> Artificial
   <220>
   <221> amorce
   <222> (1)..(20)
   <223> Amorce indirecte pour l'amplification du domaine variable de la chaîne lourde du Fab mutant F32-H
   <400> 24
   tacaccagcg gtagttaatg 20
<210> 25
   <211> 18
   <212> DNA
   <213> Artificial
   <220>
   <221> amorce
   <222> (1)..(18)
   <223> Amorce directe pour l'amplification du domaine variable de la chaîne lourde du Fab mutant H35-H
   <400> 25
   ggtgtagcct gggttcgc 18
<210> 26
   <211> 20
   <212> DNA
   <213> Artificial
   <220>
   <221> amorce
   <222> (1)..(20)
   <223> Amorce indirecte pour l'amplification du domaine variable de la chaîne lourde du Fab mutant H35-H
   <400> 26
   aacccaggct acaccaaagg 20
<210> 27
   <211> 20
   <212> DNA
   <213> Artificial
   <220>
   <221> amorce
   <222> (1)..(20)
   <223> Amorce directe pour l'amplification du domaine variable de la chaîne lourde du Fab mutant W36-H.
   <400> 27
   gtacacgcgg ttcgccagtc 20
   <210> 28
   <211> 23
   <212> DNA
   <213> Artificial
   <220>
   <221> amorce
   <222> (1)..(23)
   <223> Amorce indirecte pour l'amplification du domaine variable de la chaîne lourde du Fab mutant W36-H.
   <400> 28
   gcgaaccgcg tgtacaccaa agg 23
<210> 29
   <211> 20
   <212> DNA
   <213> Artificial
   <220>
   <221> amorce
   <222> (1)..(20)
   <223> Amorce directe pour l'amplification du domaine variable de la chaîne lourde du Fab mutant R38-H
<400> 29
   tgggttgccc agtctccagg 20
<210> 30
   <211> 17
   <212> DNA
   <213> Artificial
   <220>
   <221> amorce
   <222> (1)..(17)
   <223> Amorce indirecte pour l'amplification du domaine variable de la chaîne lourde du Fab mutant R38-H.
   <400> 30
   tggagactgg gcaaccc 17
<210> 31
   <211> 20
   <212> DNA
   <213> Artificial
   <220>
   <221> amorce
   <222> (1)..(20)
   <223> Amorce directe pour l'amplification du domaine variable de la chaîne lourde du Fab mutant W52-H.
   <400> 31
   ggagtgatag cgagaagtgg 20
<210> 32
   <211> 19
   <212> DNA
   <213> Artificial
   <220>
   <221> amorce
   <222> (1)..(19)
   <223> Amorce indirecte pour l'amplification du domaine variable de la chaîne lourde du Fab mutant W52-H.
   <400> 32
   acttctcgct atcactccc 19
<210> 33
   <211> 23
   <212> DNA
   <213> Artificial
   <220>
   <221> amorce
   <222> (1)..(23)
   <223> Amorce directe pour l'amplification du domaine variable de la chaîne lourde du Fab mutant R53-H.
   <400> 33
   gtgatatggg caagtggaat cac 23
<210> 34
   <211> 21
   <212> DNA
   <213> Artificial
   <220>
   <221> amorce
   <222> (1)..(21)
   <223> Amorce indirecte pour l'amplification du domaine variable de la chaîne lourde du Fab mutant R53-H.
<400> 34
   tccacttgcc catatcactc c 21
<210> 35
   <211> 21
   <212> DNA
   <213> Artificial
   <220>
   <221> amorce
   <222> (1)..(21)
   <223> Amorce directe pour l'amplification du domaine variable de la chaîne lourde du Fab mutant V61-H.
<400> 35
   tacaatgcac ctttcatgtc c 21
<210> 36
   <211> 22
   <212> DNA
   <213> Artificial
   <220>
   <221> amorce
   <222> (1)..(22)
   <223> Amorce indirecte pour l'amplification du domaine variable de la chaîne lourde du Fab mutant V61-H.
   <400> 36
   gaaaggtgca ttgtagtctg tg 22
<210> 37
   <211> 18
   <212> DNA
   <213> Artificial
   <220>
   <221> amorce
   <222> (1)..(18)
   <223> Amorce directe pour l'amplification du domaine variable de la chaîne lourde du Fab mutant N95-H.
   <400> 37
   gccaaagctg atcctggg 18
<210> 38
   <211> 18
   <212> DNA
   <213> Artificial
   <220>
   <221> amorce
   <222> (1)..(18)
   <223> Amorce indirecte pour l'amplification du domaine variable de la chaîne lourde du Fab mutant N95-H.
   <400> 38
   aggatcagct ttggcaca 18
<210> 39
   <211> 21
   <212> DNA
   <213> Artificial
   <220>
   <221> amorce
   <222> (1)..(21)
   <223> Amorce directe pour l'amplification du domaine variable de la chaîne lourde du Fab mutant F100K-H.
   <400> 39
   acaggcgctg cttactgggg c 21
<210> 40
   <211> 21
   <212> DNA
   <213> Artificial
<220>
   <221> amorce
   <222> (1)..(21)
   <223> Amorce indirecte pour l'amplification du domaine variable de la chaîne lourde du Fab mutant F100K-H..
   <400> 40
   gtaagcagcg cctgtcccag g 21
<210> 41
   <211> 24
   <212> DNA
   <213> Artificial
   <220>
   <221> amorce
   <222> (1)..(24)
   <223> Amorce directe pour l'amplification du domaine variable de la chaîne lourde du Fab mutant Y102-H.
<400> 41
   ggctttgctg cctggggcca aggg 24
<210> 42
   <211> 24
   <212> DNA
   <213> Artificial
   <220>
   <221> amorce
   <222> (1)..(24)
   <223> Amorce indirecte pour l'amplification du domaine variable de la chaîne lourde du Fab mutant Y102-H.
   <400> 42
   gccccaggca gcaaagcctg tccc 24
<210> 43
   <211> 21
   <212> DNA
   <213> Artificial
   <220>
   <221> amorce
   <222> (1)..(21)
   <223> Amorce directe pour l'amplification du domaine variable de la chaîne lourde du Fab mutant W103-H.
   <400> 43
   tttgcttacg cgggccaagg g 21
<210> 44
   <211> 21
   <212> DNA
   <213> Artificial
   <220>
   <221> amorce
   <222> (1)..(21)
   <223> Amorce indirecte pour l'amplification du domaine variable de la chaîne lourde du Fab mutant W103-H.
   <400> 44
   ttggcccgcg taagcaaagc c 21
<210> 45
   <211> 18
   <212> DNA
   <213> Artificial
   <220>
   <221> amorce
   <222> (1)..(18)
   <223> Amorce directe pour l'amplification du domaine variable de la chaîne lourde du Fab mutant Y32-L.
   <400> 45
   tacagtgctt tagcatgg 18
<210> 46
   <211> 24
   <212> DNA
   <213> Artificial
   <220>
   <221> amorce
   <222> (1)..(24)
   <223> Amorce indirecte pour l'amplification du domaine variable de la chaîne lourde du Fab mutant Y32-L.
   <400> 46
   tgctaaagca ctgtaaatat tctc 24
<210> 47
   <211> 18
   <212> DNA
   <213> Artificial
   <220>
   <221> amore
   <222> (1)..(18)
   <223> Amorce directe pour l'amplification du domaine variable de la chaîne lourde du Fab mutant W35-L.
   <400> 47
   ttagcagcgt atcagcag 18
<210> 48
   <211> 20
   <212> DNA
   <213> Artificial <220>
   <221> amorce
   <222> (1)..(20)
   <223> Amorce indirecte pour l'amplification du domaine variable de la chaîne lourde du Fab mutant W35-L.
   <400> 48
   ctgatacgct gctaaataac 20
<210> 49
   <211> 21
   <212> DNA
   <213> Artificial
   <220>
   <221> amorce
   <222> (1)..(21)
   <223> Amorce directe pour l'amplification du domaine variable de la chaîne lourde du Fab mutant Y36-L.
   <400> 49
   gcatgggctc agcagaaaca g 21
<210> 50
   <211> 23
   <212> DNA
   <213> Artificial
   <220>
   <221> amorce
   <222> (1)..(23)
   <223> Amorce indirecte pour l'amplification du domaine variable de la chaîne lourde du Fab mutant Y36-L.
   <400> 50
   ctgctgagcc catgctaaat aac 23
<210> 51
   <211> 18
   <212> DNA
   <213> Artificial
   <220>
   <221> amorce
   <222> (1)..(18)
   <223> Amorce directe pour l'amplification du domaine variable de la chaîne lourde du Fab mutant T53-L.
   <400> 51
   gcaaaagcct tagcagaa 18
<210> 52
   <211> 24
   <212> DNA
   <213> Artificial
   <220>
   <221> amorce
   <222> (1)..(24)
   <223> Amorce indirecte pour l'amplification du domaine variable de la chaîne lourde du Fab mutant T53-L.
   <400> 52
   tgctaaggct tttgcatcat ggac 24
<210> 53
   <211> 23
   <212> DNA
   <213> Artificial
   <220>
   <221> amorce
   <222> (1)..(23)
   <223> Amorce directe pour l'amplification du domaine variable de la chaîne lourde du Fab mutant C88-L.
   <400> 53
   tattacgctc aacatcatta tgg 23
<210> 54
   <211> 21
   <212> DNA
   <213> Artificial
   <220>
   <221> amorce
   <222> (1)..(21)
   <223> Amorce indirecte pour l'amplification du domaine variable de la chaîne lourde du Fab mutant C88-L.
   <400> 54
   atgttgagcg taataagtcc c 21
<210> 55
   <211> 20
   <212> DNA
   <213> Artificial
   <220>
   <221> amorce
   <222> (1)..(20)
   <223> Amorce directe pour l'amplification du domaine variable de la chaîne lourde du Fab mutant H91-L.
   <400> 55
   caacatgctt atggtaatcc 20
<210> 56
   <211> 19
   <212> DNA
   <213> Artificial
<220>
   <221> amorce
   <222> (1)..(19)
   <223> Amorce indirecte pour l'amplification du domaine variable de la chaîne lourde du Fab mutant H91-L.
   <400> 56
   accataagca tgttgacag 19
<210> 57
   <211> 21
   <212> DNA
   <213> Artificial
   <220>
   <221> amorce
   <222> (1)..(21)
   <223> Amorce directe pour l'amplification du domaine variable de la chaîne lourde du Fab mutant Y92-L.
   <400> 57
   catcatgctg gtaatcctcc g 21
<210> 58
   <211> 22
   <212> DNA
   <213> artificial
   <220>
   <221> amorce
   <222> (1)..(22)
   <223> Amorce indirecte pour l'amplification du domaine variable de la chaîne lourde du Fab mutant Y92-L..
   <400> 58
   attaccagca tgatgttgac ag 22

## Description

L'invention appartient au domaine de l'immunothérapie, elle concerne de nouveaux ligands pour la molécule CD4, des fragments Fab mutants de l'anticorps anti-CD4, 13B8.2. Elle concerne aussi des compositions pharmaceutiques contenant ces ligands pour le traitement des pathologies dans lesquelles la molécule CD4 est impliquée.

Depuis l'identification de la molécule CD4 par l'anticorps W3/25 et la démonstration pour cet anticorps d'un effet biologique d'immunosuppression, on connaît que les anticorps anti-CD4 ont revêtu un intérêt clinique important. Ils sont désormais la cible de nombreuses attentions de la part de l'industrie pharmaceutique, pour laquelle, ils représentent un marché de plusieurs de dizaines de millions d'individus, regroupant des pathologies aussi variées que les désordres auto-immuns, le rejet de greffe et l'infection par VIH. La Demanderesse a maintenant conçu de nouveaux ligands dérivés d'un anticorps anti-CD4, l'anticorps monoclonal murin 13B8.2, qui présentent un intérêt thérapeutique certain.

A l'heure actuelle, il existe environ une centaine d'anticorps anti-CD4 décrits pour la plupart comme ayant une activité immunosuppressive. Cependant la majorité de ces anticorps ne sont utilisés qu'en tant que réactifs immunologiques (ELISA, cytométrie de flux, diagnostic, ...). Une petite dizaine d'anticorps ont été développés pour une utilisation thérapeutique mais à l'heure actuelle seuls cinq anticorps anti-CD4 sont effectivement en cours d'essais cliniques : 1) *L'anticorps OKT4a : d*'origine murine, l'anticorps OKT4a reconnaît à la surface de la molécule CD4 humaine un épitope chevauchant la région CDR2-*like* du domaine D1. 2) Le *Clenoliximab :* cet anticorps anti CDR2-*like* du domaine D1 du CD4 a été caractérisé et étudié sous le nom de Keliximab et amélioré par Newman et al. par mutation pour présenter non seulement une capacité à lier le récepteur Fc diminuée d'un facteur 1000 mais également une demi-vie améliorée de quatre à neuf jours. 3) *L'anticorps Hu5A8 :* il s'agit d'une IgG4/κ anti-domaine D2 du CD4 d'origine murine humanisée par la méthode des CDR-grafting (Boon *et al*., 2002). Cet anticorps s'est avéré avoir une forte activité anti-VIH (Burkly *et al*., 1992 ; Moore *et al*., 1992 ; Reimann *et al*., 1997), sa nature non-immunosuppressive, et l'absence d'effet nocif sur le taux de lymphocytes T CD4⁺ circulants (Reimann *et al*., 1997) font de lui un excellent candidat dans le cadre du traitement du SIDA en association ou bien chez des patients réfractaires aux thérapies actuellement proposées. 4) *L'anticorps MDX-CD4 :* Cet anticorps anti-domaine D1 du CD4 est totalement humain et n'a nécessité aucune ingénierie (Fishwild *et al*., 1996). Isolé à partir d'un hybridome murin après immunisation des souris transgéniques, il est actuellement utilisé sous une forme réexprimée en cellules CHO ce qui n'affecte en rien sa spécificité, son affinité pour la molécule CD4 ni ses caractéristiques fonctionnelles (Fishwild *et al*., 1999).5) *L'anticorps CAMPATH-9H* qui est une IgG1/κ humanisée par la méthode des CDR-grafting à partir d'un anticorps de rat (Gorman *et al*., 1991). Obtenu par immunisation de rats, cet anticorps anti-domaine D1 du CD4 a été initialement décrit comme améliorant la qualité du traitement d'une pathologie autoimmune par un autre anticorps, le CAMPATH-1H dirigé lui contre la molécule CD52. Une nouvelle ingénierie a permis de produire cet anticorps en cellules de myélome de souris non sécréteur NSO et n'induit pas de mécanisme de cytotoxicité dépendante du complément (CDC) et que très peu les mécanismes de cytotoxicité cellulaire dépendante de l'anticorps (ADCC) (Peakman *et al*., 1994).

Enfin, l'anticorps monoclonal 13B8.2 (IOT4a) a été décrit dès la fin des années 1980 comme un agent antiviral prévenant la prolifération de VIH. Ces données l'ont immédiatement conduit en phase I de test clinique dans un essais portant sur sept patients atteints de SIDA (Dhiver *et al*., 1989). Plusieurs autres essais cliniques ont été réalisés avec cet anticorps 13B8.2 (Schedel *et al*., 1993 ; Deckert *et al*., 1996 ; Schedel *et al*., 1999). Toutes ces études ont confirmé le bénéfice clinique de l'administration de l'anticorps sur la progression de la maladie. Les travaux réalisés par le groupe de C. Devaux ont permis de déterminer le mécanisme d'action de l'anticorps 13B8.2 (pour revue, Briant et Devaux, 2000).

Il a été démontré l'existence d'une association entre son activité antivirale et une inhibition de la prolifération du VIH consécutive à l'inactivation des cascades de signalisations intracellulaires permettant normalement l'induction de l'expression du génome viral.

Cette activité antivirale pourrait être la conséquence de plusieurs facteurs comme l'induction d'un signal négatif ne dépendant ni de la p56*^{lck}* ni des corécepteurs de VIH ou l'inhibition d'un mécanisme fonctionnel impliquant la dimérisation/oligomérisation de la molécule CD4. Dans ces deux cas, l'anticorps 13B8.2 inhibe des cascades de signalisation impliquant la voie des MAPkinases, voies qui aboutissent normalement à la translocation nucléaire du facteur de transcription NF-κB.

Cet anticorps monoclonal 13B8.2 a été décrit comme dirigé comme la boucle CDR3-*like* du domaine D1 de la molécule CD4 (Sattentau *et al*., 1989 ; Corbeau *et al*., 1993 ; Houlgatte *et al*., 1994).

Les premiers essais cliniques entrepris avec l'anticorps 13B8.2 présageaient du potentiel thérapeutique de cette molécule. Cependant, son développement vers un produit d'intérêt pharmaceutique se heurtait aux nombreux problèmes inhérents à la structure et à l'origine murine de l'anticorps. En effet, ces premiers essais cliniques, portant sur des patients atteints de SIDA et incluant l'anticorps 13B8.2 dans sa version murine originale, ont démontré l'induction d'une réponse HAMA, qui même relativement faible, interférait tout de même avec l'efficacité thérapeutique de la molécule (Dhiver *et al*., 1989 ; Deckert *et al*., 1996 ; Schedel *et al*., 1999).

De façon à limiter l'immunogénicité de cette molécule et à développer un produit d'intérêt thérapeutique, un fragment Fab chimérique recombinant de l'anticorps 13B8.2 a été conçu et préparé, après isolation et séquençage des domaines V de l'anticorps 13B8.2 (Chardès *et al*., 1999), par expression, par le système baculovirus/cellule d'insecte, pour présenter les domaines V des chaînes lourdes et légères de l'anticorps parental murin fusionnés respectivement avec des domaines CH₁-γ1 et Cκ humains.

Il a été démontré que le fragment Fab chimérique recombinant de l'anticorps anti-CD4 13B8.2 est capable de lier CD4 avec la même spécificité épitopique que l'anticorps parental. De plus, ce fragment reproduit les propriétés biologiques de l'anticorps 13B8.2 d'un point de vue (1) antiviral, inhibition de l'activation du promoteur VIH et de l'activité réverse-transcriptase, et (2) immunosuppresseur, inhibition de l'activation cellulaire suite à la présentation d'antigène et de réactions mixtes lymphocytaires.

Cette fonctionnalité avait déjà été démontrée pour le fragment Fab chimique de l'anticorps (Benkirane *et al*., 1995). La chimérisation n'affecte donc pas sa capacité à inhiber la prolifération virale, même si on note une efficacité relativement inférieure.

L'évaluation des propriétés biologiques du fragment Fab recombinant, montre que ce fragment Fab présente une activité similaire à celle de l'anticorps parental tant en termes d'inhibition de l'activation du promoteur VIH que dans la capacité à inhiber la sécrétion d'IL-2 d'un lymphocyte T CD4⁺ en réponse à la présentation d'antigène (The chimeric mouse-human anti-CD4 Fab 13B8.2 expressed in baculovirus inhibits both antigen présentation and HIV-1 promoter activation. Bès C. et al. Human Antibodies 10 (2001) 67-76.)

Le fragment Fab chimérique recombinant de l'anticorps 13B8.2 possède le double avantage d'être de plus petite taille qu'un anticorps complet, ce qui améliorerait ses constantes pharmacocinétiques et lui permettrait dans un sens d'échapper au système immunitaire, mais aussi d'être un bon immunosuppresseur, ce qui expliquerait l'absence de réponse de type HAMA remarquée lors des phases cliniques préliminaires effectuées avec l'anticorps complet sous sa forme murine.

Lors des récents travaux, la Demanderesse a pu identifier des résidus critiques du paratope de l'anticorps 13B8.2 impliqués dans la liaison à la molécule CD4, qui supposent un progrès dans la conception de molécules anti-CD4 de nouvelle génération. En effet, sur la base des peptides immunoréactifs avec la molécule CD4, préalablement caractérisés (Bès C. et al. 2001b).

Une telle étude d'un paratope d'un anticorps est effectuée en utilisant des peptides chevauchant les régions variables du fragment Fab avec un antigène marqué.

Cette approche est inhabituelle, car il est communément accepté que pour qu'un anticorps puisse reconnaître l'antigène contre lequel il est dirigé, la structure tridimensionnelle de son paratope doit être conservée.

La Demanderesse a maintenant appliqué cette approche aux régions variables du fragment Fab de l'anticorps chimère 13B8.2.

Elle a ainsi pu identifier des mutations qui, bien qu'elles affectent ou pas les résidus critiques dans l'interaction anticorps/ligand des fragments Fab chimériques recombinants de l'anticorps 13B8.2 de seconde génération, améliorent les propriétés biologiques desdits fragments.

Les travaux de la Demanderesse, réalisés dans le cadre de la présente invention, confirment les résultats prédictifs d'une telle approche, en effet, les résultats expérimentaux obtenus par des techniques de mutagenèse dirigée, prouvent que la méthode Spot de multisynthèse parallèle de peptides sur support de cellulose (Frank R, Tetrahedron, 1992, 48 :9217-9232) appliquée au paratope de l'anticorps 13B8.2 permet d'identifier des résidus critiques impliqués dans l'activité biologique dudit anticorps.

D'une part, l'obtention de molécules dérivées d'un anticorps plus actives présente un intérêt clinique en termes de minimisation des doses à administrer assurant un meilleur confort pour le clinicien mais aussi, et surtout, pour le patient.

D'autre part, la complémentarité de ces diverses molécules Fab présente un intérêt clinique en cas d'échappement thérapeutique vis-à-vis de l'une d'entre elles.

La Demanderesse a donc conçu des nouveaux fragments Fab mutants dérivés de l'anticorps monoclonal chimère humanisé 13B8.2 ayant des propriétés immunosuppressives.

A partir de l'hybridome murin 13B8.2, les gènes codant pour les chaînes lourdes et légères de l'anticorps 13B8.2 ont été amplifiés par une réaction de polymérisation en chaîne (PCR) en utilisant des jeux d'oligonucléotides s'hybridant dans les séquences signal des immunoglobulines. (Chardès et al. FEBS Lett 452 : 386-394, 1999).

Le clonage des régions variables de l'anticorps 13B8.2 qui utilise les gènes VH2-DQ52-JH3 et Vk12/13-Jk2 a été réalisé dans un premier temps, puis les séquences complètes des régions VH et Vk de l'anticorps 13B8.2 ont été réalisées.

Un fragment Fab humanisé de l'anticorps 13B8.2 anti-CD4 humanisé ayant les régions variables clonées de l'hybridome murin 13B8.2 et des régions constantes d'immunoglobulines humaines a ensuite été obtenu par génétique moléculaire dans un système d'expression baculovirus (Cérutti M. et al., Protéine Performance, 1995, demande de brevet, n°FR95/00110 ; INRA/CNRS, 1997 WO 95/20672 ; Poul M-A. et al., Immunotechnology, 1995,1 :189-196).

A partir de données Alascan SPOT, obtenues par remplacement d'un acide aminé dans une position donnée dans la séquence d'un peptide capable de lier la molécule CD4 par l'acide aminé alanine, la Demanderesse a pu déterminer quels sont les résidus du fragment Fab de l'anticorps 13B8.2 ayant une contribution particulièrement importante pour leur liaison à la molécule CD4.

Sur la base des résultats d'Alascan, une série de 16 Fab mutants de l'anticorps anti-CD4 humanisé 13B8.2 a été produite. Pour cela, 16 résidus issus des régions hypervariables CDR1/CDR2/CDR3-VH de la région variable de la chaîne lourde et CDR1/CDR2-Vκ de la région variable de la chaîne légère de l'anticorps 13B8.2 ont été mutés successivement d'une alanine afin d'évaluer la nature des résidus contributeurs pour la liaison à la molécule CD4.

Les fragments Fab mutants ont été produits dans le système baculovirus/cellules d'insecte (Cérutti M. et al., 1995 brevet, Protéine Performance n°FR 95/00110 INRA/CNRS, 1997 WO 95/20672 Poul M-A. et al., Immunotechnology, 1995, 1 :189-196). La qualité des mutants a été évaluée par ELISA et Western Blot. Il a été trouvé que huit résidus (H35, W52, R53, F100κ, W103 du VH et Y32, Y36 et H91 du Vκ) sont fortement contributeurs, étant donné qu'une perte de liaison à la molécule CD4 desdits fragments Fab mutants est observée, tant par des techniques ELISA, Tri par fluorescence (FACS) ou BIAcore.

Trois résidus (R38 du VH, W35 et Y92 du Vκ) sont moyennement contributeurs et il a été démontré que cinq autres résidus (F32, W36, C92, Y102 du VH et C88 du Vκ) ne sont pas contributeurs à la liaison du fragment Fab avec la molécule CD4.

Les fragments Fab mutants présentant des mutations sur les acides aminés définies comme contribuant à la liaison avec la molécule CD4 présentent également une perte d'efficacité biologique, telle que l'activité d'inhibition du promoteur VIH, ou l'activité d'inhibition de présentation de l'antigène.

La modélisation moléculaire du paratope 13B8.2 a été réalisée et constitue un élément de compréhension supplémentaire à l'interaction avec la molécule CD4.

Le rôle des résidus chargés positivement R53 du VH et surtout H35 du VH et H91 du Vκ semble fondamental, probablement en permettant la liaison à la région homologue du CDR3-*like* de la molécule CD4, en effet, cette région de la molécule CD4 est chargée négativement avec les acides aminés E87 et D88.

Cette analyse est confortée par le fait qu'un gradient de pH de 6 à 8, diminuant la charge positive des acides aminés Histidine de 50% à 5%, entraîne une diminution de la liaison de l'ordre de 25% en ELISA.

En conclusion, cinq Fab mutants de l'anticorps anti-CD4 humanisé 13B8.2 conservent la capacité de liaison à la molécule CD4 et les propriétés immunosuppressives de l'anticorps parental 13B8.2.

L'invention a donc pour objet un Fragment Fab mutant de l'anticorps anti-CD4 13B8.2, liant la molécule CD4 et comportant une mutation d'au moins un résidu F32, W36, C92, Y102 dans le domaine variable VH de la chaîne lourde et/ou C88 dans le domaine variable Vκ de la chaîne légère.

De préférence, les fragments Fab mutants de l'anticorps 13B8.2 de l'invention présentent une constante d'affinité pour la molécule CD4 au moins égale à celle du Fab sauvage.

Un fragment Fab mutant de l'anticorps anti-CD4 13B8.2 comportant une mutation d'au moins un résidu dans une position située dans les régions 31-41, 49-57, 61-70 ou 90-103 du domaine variable V_{H} de la chaîne lourde ou dans une position située dans les régions 19-26, 32-40 et 85-96 du domaine variable Vκ de la chaîne légère est également décrit.

De préférence, le fragment fab mutant de l'invention comporte une mutation C88.L dans le domaine variable Vκ, ou une mutation F32-H, W36-H, C92-H ou Y102-H dans le domaine variable V_{H}.

Un fragment Fab mutant, choisi parmi le groupe comprenant les Fabs mutants C88-L, dont la région variable Vκ présente une mutation du résidu Cystéine en position 88 par une Alanine et est identifiée par la séquence SEQ ID No: 16 dans la liste de séquences en annexe ; F32-H, dont la région variable VH présente une mutation du résidu phénylalanine en position 32 par un résidu alanine et est identifiée par la séquence SEQ ID No: 3 dans la liste de séquences en annexe ; W36-H, dont la région variable VH présente une mutation du résidu tryptophane en position 36 par un résidu alanine et est identifiée par la séquence SEQ ID No: 5 dans la liste de séquences en annexe ; C92-H dont la région variable VH présente une mutation du résidu cystéine en position 92 par un résidu alanine et est identifiée par la séquence SEQ ID No: 9 dans la liste de séquences en annexe et Y102-H, dont la région variable VH présente une mutation du résidu tyrosine en position 102 par un résidu alanine et est identifiée par la séquence SEQ ID No: 11 dans la liste de séquences en annexe, est également décrit.

L'invention concerne des compositions pharmaceutiques comprenant, à titre de principe actif, une dose efficace d'au moins un des fragments Fab mutants, de l'invention éventuellement en présence d'un excipient approprié.

Des compositions pharmaceutiques dans lesquelles le Fab mutant peut-être utilisé à une concentration comprise entre 1 et 50 mg, de préférence entre 5 et 10 mg sont décrites.

Dans une composition pharmaceutique selon l'invention le Fab mutant est utilisé à une concentration comprise entre 0,01 mg/kg et 2 mg/kg, de préférence entre 0,1 et 0,4 mg/kg en poids du patient à traiter.

Les compositions pharmaceutiques de l'invention sont particulièrement utiles pour le traitement des pathologies autoimmunes, notamment la polyarthrite rhumatoïde, mais aussi le psoriasis ou le lupus érythémateux.

Les compositions pharmaceutiques de l'invention peuvent être utilisées en combinaison avec des préparations comprenant des ligands anti-TNF.

Les compositions pharmaceutiques de l'invention sont particulièrement utiles pour le traitement ou la prévention de réactions d'intolérance immunologique.

Les compositions pharmaceutiques de l'invention sont également utiles pour le traitement des réactions déclenchées chez un patient à la suite d'une transplantation d'un organe, pour diminuer, voire éliminer les réactions du type greffon vers l'hôte, afin d'améliorer la tolérance du greffon.

Les compositions pharmaceutiques de l'invention sont également utiles pour le traitement de cancers impliquant la molécule CD4, tels que les lymphomes CD4+ ou le lymphome de Cesari.

Enfin une utilisation tout particulièrement indiqué des compositions pharmaceutiques de l'invention est celle du traitement du SIDA, comme la prévention de la transmission virale mère-enfant, voire dans le cadre d'un traitement préventif lors d'une contamination accidentelle.

Les fragments Fab mutants de l'anticorps anti-CD4 chimère 13B8.2 sont particulièrement adaptés pour une thérapie immunosuppressive et permettent, notamment d'éviter l'échappement au traitement car ils n'induisent pas de réponse anti-idiotypique.

Ainsi une composition pharmaceutique selon l'invention est utile pour la préparation d'un médicament destiné à la prévention ou le traitement d'immunodéficiences liées à une infection virale.

L'invention est décrite ci-après par la description des travaux expérimentaux effectués par la Demanderesse afin de préparer, purifier et identifier les Fabs mutants de l'anticorps 13B8.2, de comparer leur comportement vis-à-vis de leur ligand, la molécule CD4, par rapport à celui du Fab sauvage, et de sélectionner ainsi ceux présentant les meilleures activités biologiques.

Elle est illustrée par les figures en annexe dans lesquelles :
La figure 1 montre la détermination des résidus contribuant à la liaison au CD4 du paratope de l'anticorps 13B8.2 par balayage Spot alanine.
   - la figure 1A illustre le balayage d'une membrane avec une sonde CD4, correspondant au peptide 1 GVIWRS et au peptide 2 WRSGIT couvrant les résidus 49-57 de la région la région CDR-H2 selon la numérotation de Kabat et leurs sets respectifs d'hexapeptides analogues, avec l'alanine.
   - la figure 1B illustre l'analyse quantitative des réactivités Spot des peptides 1 et 2 et de leurs analogues alanine respectifs couvrant les résidus 49-57 de la région CDR-H2 région. Chaque barre représente la réactivité d'un hexapeptide dont la séquence comprend un résidu Ala à la place de l'acide aminé indiqué.
   - la figure 1C montre les résidus contributeurs de Spot de chacune des régions CDR des régions variables des chaînes lourdes et légères, mesurés en pourcentage d'inhibition de la liaison à la molécule CD4 (barre verticale). L'identification des CDR effectuée selon Kabat (boîtes pleines) et IMGT (Boîtes ombrées) est indiquée.
La figure 2 illustre la caractérisation des Fab recombinants ayant des résidus mutés par des alanines, après une immunopurification sur de la protéine G à partir des surnageants de baculovirus.
   - la figure 2A illustre l'analyse par ELISA des mutants Y36-L, C88-L, F32-H, H35-H, W52-H et R53-H par rapport à une courbe standard d'immunoglobuline.
   - la figure 2B montre l'analyse par Western blot de la bande de 50 Kda desdits Fabs révélée avec un anticorps anti-chaîne kappa conjugué à la peroxydase
La figure 3 montre les courbes de liaison obtenues par une méthode ELISA des Fabs mutants de l'anticorps 13B8.2 sur de la molécule CD4 absorbée par rapport à celles obtenues avec le Fab sauvage et un contrôle Fab1C10, chaque valeur représentant la moyenne ± S.D des déterminations par triplicatas et sont représentatives de trois expériences différentes.
La figure 4 montre les analyses par cytométrie de flux de la liaison à des cellules T A2.01/CD4 de chacun des Fabs recombinants de l'anticorps 13B8.2 versus le Fab sauvage et un Fab 1C10 contrôle. Les résultats sont représentatifs de deux expériences différentes. La concentration en anticorps y est indiquée.
La figure 5 montre l'inhibition de la sécrétion d'Il2 par les cellules T pdb 10f, sensibilisées avec les cellules présentatrices d'antigène EBV-Lu stimulées avec le peptide pep24 et co-cultivées avec les Fabs recombinants de l'anticorps 13B8.2.
   - la figure 5A montre le pourcentage dose-réponse de l'inhibition de la sécrétion d'Il2 à des concentrations différentes du Fab sauvage par rapport au Fab contrôle 1C10.
   - la figure 5B montre le pourcentage d'inhibition de la sécrétion d'Il2 pour chacun des Fab recombinants mutants (nd= non-déterminé). La moyenne des absorbances à 450 nm varie de 0,015 pour les cellules pdb 10F co-cultivées avec des cellules présentatrices EBV-Lu non-stimulées à 1,28 pour des cellules pdb 10F co-cultivées avec des cellules présentatrices d'anticorps EBV-Lu stimulées avec le peptide pep24. Le contrôle positif pour la sécrétion d'Il2 par incubation de l'anticorps anti-CD3 murin (Pharmingen, San Diego, CA) avec les cellules T pdb 10F produit une absorbance de 2,30.
La figure 6 montre l'inhibition de l'expression de gène de la galactosidase contrôlé par le LTR du VIH-1_{Lai} après l'incubation avec les Fabs recombinants mutants de l'anticorps 13B8.2.
   - la figure 6A montre l'inhibition de l'expression du gène de la béta-galactosidase dans des cellules HeLa P4 infectées par le virus VIH-1_{Lai} cultivées en présence de différentes concentrations de Fab recombinant sauvage.
   - la figure 6B montre l'inhibition de l'expression du gène de la béta-galactosidase pour chacun des Fabs recombinants mutants H91-L, F32-H, H35-H, W52-H et R53-H. La moyenne d'absorbance à 410 nm varie de 0,01 pour les cellules indicatrices non-infectées à 0,40 pour les cellules indicatrices infectées par le virus VIH-1_{Lai}.
La figure 7 montre un modèle tridimensionnel des régions variables des chaînes lourdes et légères de l'anticorps 13B.2 généré par le logiciel AbM, basé sur une modélisation d'homologie (vue frontale). Le modèle informatique est montré comme une trace Cα avec les chaînes latérales pour chaque résidu contributeur sélectionné par Spot pour le paratope de l'anticorps 13B8 .2

Les résidus SCR liant CD4, comme confirmé par mutagenèse dirigée sont identifiées en gris foncé (red color) alors que ceux non impliqués dans la liaison au CD4, comme défini par mutagenèse, sont en gris clair (blue) cette figure a été obtenue avec le logiciel de visualisation Swiss pdb Viewer.

La figure 8 montre le schéma de la méthode utilisée pour amplifier les séquences codant les domaines variables des chaînes lourde et légère des fragments Fab mutants de l'anticorps 13B8.2. Ces séquences ont été obtenues par PCR chevauchant.

### MATERIEL ET METHODE

### Réactifs, lignées cellulaires et vecteurs.

Le plasmide pMC7-T4 codant pour la totalité de la séquence de l'ADNc de la molécule CD4 [Maddon, 1987], a été utilisé. (Comme cela a été décrit [Bès, 2001a, 2001b], le baculovirus ayant le CD4 inséré ont été construits et utilisés postérieurement pour la production de CD4 recombinant humain soluble. Pour les analyses SPOT, du CD4 humain recombinant (Repligen Inc, Needham, MA) a été biotinylé en utilisant des réactifs commerciaux (Amersham Pharmacia Biotech, Cleveland, OH) selon les instructions du fabricant.

La lignée de l'hybridome murin qui produit l'anticorps monoclonal 13B8.2 (IgG1/κ) [Dhiver, 1989; Corbeau, 1993] a été obtenue auprès du D. Olive et C. Mawas (INSERM U119, Marseille, France).

La lignée lymphoblastoïde B EBV-Lu exprimant les molécules HLA DR5,6, DRB52, DQ6,7 et A2 et la lignée murine T pdb10F exprimant le CD4 humain et le peptide pep24 (PAGFAILKCNNKTFNY) spécifique du TCR chimérique [Manca, 1996] a été obtenu auprès du Pr. De Berardinis (Consiglio Nazionale delle Ricerche, Napoly, Italy).

La lignée de cellules indicatrices HeLa P4 ayant le LTR β-galactosidase de VIH-1 [Briant, 1998] ont été obtenues auprès de O. Schwartz (Institut Pasteur, Paris, France). La lignée cellulaire T A2.1/CD4 [Poulin, 1991] exprimant le CD4 humain sauvage a été obtenue auprès du D. Littman (New York, NY).

Analyse par alascan Spot pour identifier les résidus contributeurs des séquences immunoréactives de l'anticorps 13B8.2 vis-à-vis du CD4.

Le protocole général pour la synthèse de peptides Spot sur des membranes de cellulose utilisée et celui décrit [Monnet, 1999; Laune, 2002]..

A partir de 202 dodecapeptides chevauchants dont le cadre est déplacé d'un seul résidu, correspondant à la séquence d'acides aminés déduite des régions variables de l'anticorps 13B8.2, l'immunoréactivité anti-CD4 a été observée préalablement [Bès, 2001b] pour des peptides incluant les séquences 31-41, 49-70 et 90-103 de la chaîne lourde et les séquences 19-26, 32-40 et 85-96 de la chaîne légère (selon la numérotation de Kabat).

Dix-sept hexapeptides, couvrant lesdites séquences immunoreactives et six analogues de chaque hexapeptide ont été synthétisés par la méthode Spot.

La réactivité antigénique des peptides liés à la membrane de cellulose a été testée avec du CD4 biotinylé (1µg/ml) dans des conditions qui conduisent à l'obtention d'un précipité bleu pour les spots réactifs selon décrit dans [Bès, 2001b]. La réactivité des spots a été évaluée par balayage de la membrane et mesure des intensités des spots avec le logiciel NIH image 1.61. Les spots des résidus contributeurs (SCR) du paratope de l'anticorps 13B8.2 ont été identifiés sur la base d'une diminution égale ou supérieur à 50% de la capacité de liaison pour l'antigène par rapport à celle de la séquence peptidique non-modifiée.

### Construction de baculovirus recombinants produisant des Fabs sauvages ou mutants de l'anticorps 13B8.2.

Les procédures générales concernant le clonage et le séquençage des régions variables de l'anticorps 13B8.2 ont été réalisées selon décrit dans [Chardès, 1999; Bès, 2001a, 2001b]. La mutagenèse dirigée des gènes des chaînes lourdes et légères de l'anticorps 13B8.2 ont été effectuées par PCR chevauchant [Ho, 1989]. Les seize positions ayant été identifiées comme des résidus contributeurs de spot (SCR) par balayage d'alanine et deux résidus additionnels (T53 de la chaîne légère et V61 de la chaîne lourde) comme contrôle, ont été mutés par une alanine.

Chaque mutant a été vérifié par séquençage.

Pour la préparation des gènes des chaînes lourdes des fragments de la région variable de la chaîne lourde linéarisée *PstI*/*SacI* ont été clonés dans la cassette du plasmide de vecteur de transfert pBHuFdγ₁ qui contient pre-installé en amont le premier domaine de la chaîne lourde Cγ₁ (Fdγ₁) permettant l'insertion et l'expression de la chaîne lourde de l'anticorps 13B8.2 sous le contrôle du promoteur polyhedrine [Poul, 1995; Bès, 2001a].

Pour la préparation des gènes des chaînes légères, les fragments de la région variable de la chaîne légère, linéarisés *XhoI*/*KpnI*, ont été clonés dans la cassette du plasmide du vecteur de transfert pBHuCκ qui contient pre-installé en amont, le gène Cκ [Poul, 1995; Bès, 2001a], permettant l'insertion et l'expression de la chaîne légère de l'anticorps 13B8.2 sous le contrôle du promoteur p10.

Une procédure de recombinaison en deux étapes, [Poul, 1995; Bès, 2001a] est effectuée pour construire les baculovirus recombinants exprimant les deux chaînes lourdes et légères des Fab sauvage et mutants de l'anticorps 13B8.2. Un Fab contrôle, non relevant, le Fab 1C10 anti-digoxine est exprimé de manière similaire dans le système baculovirus/cellule d'insecte.

### Les amorces "13B8.2"

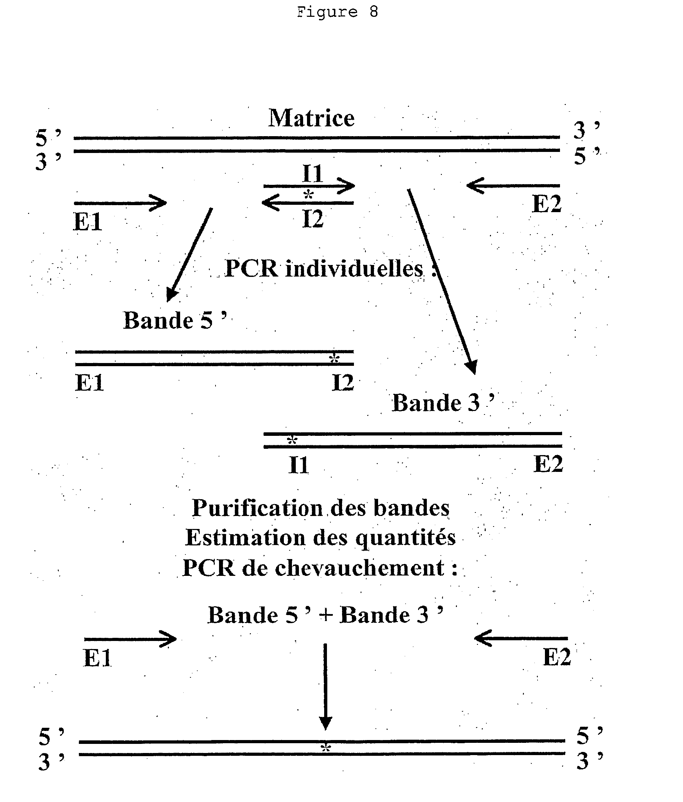

Ces amorces ont été utilisées pour amplifier les séquences codant les domaines variables des chaînes lourde et légère des fragments Fab mutants de l'anticorps 13B8.2. Ces séquences ont été obtenues par PCR chevauchant (Figure 8).

Les amorces p119F et p119R correspondent respectivement aux amorces E1 et E2 dans le cadre de l'amplification de la chaîne lourde, les amorces p116F et p116R correspondent respectivement aux amorces E1 et E2 dans le cadre de l'amplification de la chaîne légère.

Les autres amorces correspondent aux amorces I1 et I2 suivant la nomenclature : "chaîne (H ou L) " "position mutée""1 ou 2", par exemple, VhF32F et VhF32R correspondent respectivement aux amorces I1 et I2 utilisées pour muter en alanine le résidu F32 (Phe32) de la chaîne lourde.

**Tableau I**

| Amorces utilisées pour les constructions Fab 13B8.2 mutants | |
|---|---|
| **NOM** | **SEQUENCE** |
| p119R | 5' ATC CGG AAC AAT GTC GCC GG 3' |
| P119F | 5' CAT CAC TTA CAA CAA GGG GG 3' |
| p116R | 5' TAT CAG CCC CAG CGT TGC 3' |
| p116F | 5' CTG CGA GCA GTT GTT TGT 3' |
| VhF32F | 5' ACT ACC GCT GGT GTA CAC TGG 3' |
| VhF32R | 5' TAC ACC AGC GGT AGT TAA TG 3' |
| VhH35F | 5' GGT GTA GCC TGG GTT CGC 3' |
| VhH35R | 5' AAC CCA GGC TAC ACC AAA GG 3' |
| VhW36F | 5' GTA CAC GCG GTT CGC CAG TC 3' |
| VhW36R | 5'GCG AAC CGC GTG TAC ACC AAA GG 3' |
| VhR38F | 5' TGG GTT GCC CAG TCT CCA GG 3' |
| VhR38R | 5' TGG AGA CTG GGC AAC CC 3' |
| VhW52F | 5' GGA GTG ATA GCG AGA AGT GG 3' |
| VhW52R | 5' ACT TCT CGC TAT CAC TCC C 3' |
| VhR53F | 5' GTG ATA TGG GCA AGT GGA ATC AC 3' |
| VhR53R | 5' TCC ACT TGC CCA TAT CAC TCC 3' |
| VhV61F | 5' TAC AAT GCA CCT TTC ATG TCC 3' |
| VhV61R | 5' GAA AGG TGC ATT GTA GTC TGT G 3' |
| VhN95F | 5' GCC AAA GCT GAT CCT GGG 3' |
| VhN95R | 5' AGG ATC AGC TTT GGC ACA 3' |
| VhF100KF | 5' ACA GGC GCT GCT TAC TGG GGC 3' |
| VhF100KR | 5' GTA AGC AGC GCC TGT CCC AGG 3' |
| VhY102F | |
| VhY102R | |
| VhW103F | 5' TTT GCT TAC GCG GGC CAA GGG 3' |
| VhW103R | 5' TTG GCC CGC GTA AGC AAA GCC 3' |
| VlY32F | 5' TAC AGT GCT TTA GCA TGG 3' |
| VlY32R | |
| VlW35F | 5' TTA GCA GCG TAT CAG CAG 3' |
| VlW35R | 5' CTG ATA CGC TGC TAA ATA AC 3' |
| VlY36F | 5' GCA TGG GCT CAG CAG AAA CAG 3' |
| VlY36R | 5' CTG CTG AGC CCA TGC TAA ATA AC 3' |
| VlT53F | 5' GCA AAA GCC TTA GCA GAA 3' |
| VlT53R | |
| VlC88F | 5' TAT TAC GCT CAA CAT CAT TAT GG 3' |
| VlC88R | 5' ATG TTG AGC GTA ATA AGT CCC 3' |
| VlH91F | 5' CAA CAT GCT TAT GGT AAT CC 3' |
| VlH91R | 5' ACC ATA AGC ATG TTG ACA G 3' |
| VlY92F | 5' CAT CAT GCT GGT AAT CCT CCG 3' |
| VlY92R | 5' ATT ACC AGC ATG ATG TTG ACA G 3' |

### Production de Fabs recombinants, purification et caractérisation.

Chaque Fab recombinant de l'anticorps 13B8.2 a été purifié sur de la protéine G à partir de 400 ml de surnageant de cellules Sf9 *Spodoptera frugiperda* (ATCC CRL 1711) infectées avec les baculovirus recombinants, comme décrit dans [Bès, 2001a].

Les Fabs purifiés sont quantifiés par ELISA en utilisant un antisérum de mouton dirigé contre le fragment Fdγ₁ humain (The Binding Site, Birmingham, UK) en tant que réactif de capture et un anticorps dirigé contre les chaînes humaines kappa, conjugué à la peroxydase (Sigma, St Louis, MO), en tant que réactif de détection. Des échantillons des anticorps ont ensuite été testés par analyse électrophorétique et Western Blot.

### Etudes de la liaison du CD4, par les Fabs sauvages et mutants de l'anticorps 13B8.2.

Une méthode ELISA a été mise en oeuvre pour cribler initialement le Fabs de l'anticorps 13B8.2 pour leur capacité à se lier au CD4 soluble.

Une dilution 1 :500 de la fraction du CD4 exprimée par le baculovirus dans 0,1 M tampon carbonate/bicarbonate , pH 9,6 est incubée durant la nuit à 4°C surs des plaques d'immunoanalyse enzymatique de 96 puits (Nunc, Paisley, UK). Quatre lavages avec du tampon salin phosphate (PBS) 160 mM pH 7, 2, contenant 0,1% Tween 20 (PBD-T) sont effectués avant et après la saturation des microplaques avec du lait écrémé en poudre à une concentration de 1% dans du PBS-T durant une heure à 37°C.

Ensuite 100 %1 des dilutions en série deux par deux d'une solution d'anticorps à 2,5 µg/ml est ajoutée à chaque puits.

Après avoir incubé durant 2 heures et lavé dans du PBS-T, les anticorps liés sont détectés par addition de 100 µl d'une solution 1 :1000 du conjugué anti-kappa humaine -peroxydase (Sigma), suivi de l'addition du substrat de la peroxydase. L'absorbance est mesurée à 490 nm (A₄₉₀)

Les paramètres cinétiques de la liaison du CD4 au paratope des Fabs de l'anticorps 13B8.2 ont été déterminés par analyse de la résonance plasmonique de surface au moyen d'un analyseur BIAcore (BIAcore AB, Uppsala, Sweden).

Le CD4 exprimé dans le baculovirus est immobilisé de façon covalente sur un biocapteur et les Fabs recombinants dans du tampon HBS (10 mM Hepes pH 7.6, 150 mM NaCl) sont injectés à des concentrations diverses entre 5 et 20 µg/ml.

Les paramètres cinétiques ont été calculés en utilisant le logiciel d'évaluation BiaEvaluation 3.2 et la méthode globale selon [Karlsson, 1994]..

La liaison des Fab sauvage ou mutants au CD4 membranaire est évaluée par cytométrie de flux.

Des cellules T AN2.01/CD4 (1x10⁶) sont incubés avec du PBS contenant 0,2% BSA(PBS-BSA) ou avec PBS-BSA supplémenté avec chacun des Fab recombinants ou avec le Fab anti-digoxine 1C10 non relevant exprimés dans les systèmes baculovirus/cellule d'insecte. (1µg/ml).

Des expériences similaires ont été effectuées avec des cellules T AN2.01 (une lignée cellulaire T Cd4 négative). Après trois lavages avec du PBS-BSA, les anticorps liés sont révélés par incubation avec 50 µl d'une solution 1 :1000 d'un anticorps dirigé contre la chaîne légère kappa humaine conjugué à la fluorescéine (Sigma) durant une heure à 4°C). Après trois lavages subséquents, avec du PBS-BSA, l'intensité de fluorescence est mesurée dans un cytofluoromètre EPICS (Beckman-Coulter, Fullerton, CA).

### Test de sécrétion d'Il2 suite à la présentation de l'antigène.

Comme cela a été décrit [Bès, 2001a, 2001b], des cellules présentatrices d'antigène EBV-Lu pulsées avec le peptide stimulateur pep24 (10⁵ cellules/puits) ont été co-cultivées avec des cellules T répondant pdb 10F (2 x 10⁴ cellules/puits). Les Fabs sauvage ou mutants de l'anticorps 13B8.2 (20 µg/ml) ont été ajoutés aux cellules et la présentation d'antigène est effectuée durant 24 heures à 37°C. Ensuite, 100 µl du surnageant sont récupérés et testés pour la sécrétion d'Il2 en utilisant un kit ELISA commercial (Pharmingen, San Diego, CA).

### Test d'activité du promoteur VIH-1.

Des cellules indicatrices HeLa P4 (8x10⁴ cellules/ml) ont été cultivées dans du milieu supplémenté ou non avec du virus VIH-1_{Lai} infectieux en présence (20µg/ml) ou en absence des Fabs durant trois jours, récupérés et lysés. L'activité β-galactosidasique a été déterminée selon décrit préalablement en mesurant l'absorbance à 410 nm [Monnet, 1999].

### Modélisation moléculaire des régions variables de l'anticorps 13B8.2

Un modèle en trois dimensions des régions variables des chaînes lourdes et légères de l'anticorps 13B8.2 a été obtenue en utilisant le logiciel AbM (Oxford Molecular, Accelrys UK)[Rees, 1992] sur une station de travail 02 R5000 Silicon Graphics.

Les boucles L2, L3, H1 et H2-CDR ont été construits selon un cadre canonique de clase 1 et un cadre canonique de classe 2 pur la boucle L1 tel que défini par le logiciel AbM

La boucle H3 a été construite en utilisant une recherche combinée Base de données/CONGEN, un programme de recherche conformationnelle implémenté dans AbM, combiné avec une recherche dans une base de données des structures 3D.

Les hydrogènes ont été ajoutés au modèle en utilisant le logiciel SYBYL (Tripos, Inc) et le modèle a été minimisé durant 100 itérations avec le champ de force tripos et la méthode de gradient conjugué pour éliminer tous les petits conflits sériques. Les aires de surface accessibles aux solvants des acides aminés de l'anticorps 13B8.2 ont été calculées dans le modèle 3D par le logiciel SALVOL implémenté dans SYBYL.

### Mesure de l'activité réverse-transcriptase virale.

Cette méthode, utilisée dans le cadre de l'étude des propriétés antivirales du fragment Fab chimérique recombinant de l'anticorps 13B8.2, permet de mesurer une cinétique de prolifération virale en dosant dans le surnageant de culture de cellules infectées par VIH l'activité réverse-transcriptase virale.

La première étape consiste à infecter 5.10⁵ cellules CEM par échantillon à tester. Pour cela, après avoir lavé les cellules en PBS, nous les incubons pendant 30 min à 4°C en présence de 100TCIDₛ₀ de virus HIV-1_{LAI} (100 µl de virus pour 5.10⁵ cellules). Quatre étapes de lavage en milieu de culture RPMI sont alors effectuées pour ôter l'excédent de virus. Les cellules sont étalées dans des plaques p24, puis mises en culture pendant 3 jours à 37°C sous 5% CO₂ en présence des échantillons à tester sous un volume final de 1 ml. Hormis les échantillons potentiellement inhibiteurs de la prolifération virale, nous testons également l'activité réverse-transcriptase en absence d'inhibiteur, en absence de virus et en présence d'inhibiteurs connus tels que l'AZT de façon à avoir plusieurs contrôles internes.

La seconde étape consiste en l'extraction de la réverse-transcriptase virale. Cette étape est renouvelée tous les 3 jours sur une période de 15 à 20 jours. Pour cela, la plaque de culture est centrifugée pendant 3 min à 4°C à 1500 rpm de façon à récupérer le surnageant. Les cellules infectées sont remises en culture après réajustement de leur concentration à 5.10⁵ cellules/ml en présence des échantillons à tester. Le surnageant de culture est centrifugé pendant 5 min à 4°C à 95000 rpm. Le culot obtenu, contenant les particules virales produites par les cellules infectées, est incubé pendant 15 min à 4°C en présence de 15 µl de tampon de lyse (voir préparation des tampons). A ce niveau les lysats de particules virales peuvent être conservés à -80°C pour une utilisation ultérieure.

La troisième étape consiste au dosage de l'activité réverse-transcriptase. Le lysat est mis en présence de 40 µl de mélange réactionnel (voir préparation des tampons) et incubé pendant 1 h à 37°C. Le mélange réactionnel contient une matrice ARN (ARNpolyA), une amorce (oligoDT) et un mélange des quatre nucléotides dont de la déoxythymidine marquée au ³H. Cette étape permet donc de suivre l'incorporation de ³H-dTTP dans de l'ADN rétrotranscrit par la reverse transcriptase présente dans le lysat de particules virales. En effet, après arrêt de la réaction par addition de 1 ml de PPNa 0.1M en TCA 5% (pyrophosphate de sodium en acide trichloroacétique), l'ADN rétrotranscrit est précipité en présence de 200 µl d'une solution d'ADN de sperme de saumon (500 µg/ml) et de 4 ml de TCA 20% pendant 15 min à 4°C. Cette solution est filtrée sur filtre millipore 0,45µ. Les filtres sur lesquels l'ADN a été récupéré sont lavés deux fois en TCA 5% puis séchés d'abord à l'éthanol 70% puis à l'étuve pendant 10 min à 37°C. La radioactivité présente sur les filtres est alors mesurée après incorporation des filtres dans 5 ml de liquide scintillant.

La mesure de l'activité réverse-transcriptase, exprimée en cpm/ml, permet le suivi de la prolifération virale en fonction du temps en présence d'échantillons potentiellement inhibiteurs.

### Réactions mixtes lymphocytaires

Par cette approche, la Demanderesse a évalué la capacité du fragment Fab chimérique recombinant de l'anticorps 13B8.2 à inhiber la prolifération cellulaire lors de la mise en contact de deux populations lymphocytaires provenant de sang périphérique de donneurs différents.

La première étape a consisté à effectuer la réaction mixte lymphocytaire. Pour cela, des buffy-coat (préparations lymphocytaires de sang périphériques) issus de deux donneurs différents A et B sont lavés en milieu de culture RPMI (supplémentés avec 10% de sérum humain AB, d'antibiotiques pénicilline et streptomycine et de L-glutamine). Les cellules sont ensuite diluées en milieu de culture à hauteur de 1.10⁶ cellules/ml. La réaction mixte lymphocytaire se fait dans des plaques de cultures p96 selon deux schémas. Le premier, "one-way", consiste à mélanger 50 µl de la suspension cellulaire du donneur A avec 50 µl d'une suspension cellulaire de même concentration et du même donneur préalablement traitée pendant 30 min à 37°C, 5% CO₂ par la mitomycine C (concentration finale de 25 µg/ml). Ce schéma permet d'évaluer la prolifération cellulaire en réponse à des cellules "anormales". Le second, "two-way", consiste à mélanger 100 µl de suspensions cellulaires provenant des donneurs A et B non traitées à la mitomycine C. Ce schéma permet d'évaluer la prolifération cellulaire en réponse à des cellules "étrangères". Ce deuxième schéma permet en règle générale d'améliorer la sensibilité du résultat. C'est celui que nous avons préférentiellement étudié. Dans les deux cas, les cellules sont incubées pendant 5 à 7 jours en présence de 50 µl de solution d'échantillon à tester.

La seconde étape consiste au dosage de la prolifération cellulaire. Pour cela, les cellules sont incubées pendant 18 h à 37°C, 5% CO₂ en présence de 20µM de BrDU. Cette molécule est incorporée dans l'ADN des cellules en division. La plaque de culture est ensuite centrifugée de façon à éliminer le surnageant, puis séchée pendant 1 h à 37°C. Les cellules sont alors fixées pendant 30 min à 25°C par addition de 200 µl de solution de FixDenat. Après lavage de l'excédent de solution de fixation, l'incorporation du BrDU est révélée suivant le principe ELISA après incubation de 3 h à 37°C en présence d'un anticorps anti-BrDU marqué à la peroxydase et addition du substrat colorant. La mesure de l'absorbance est effectuée à 450nm.

La prolifération cellulaire est alors exprimée en unités d'absorbance en fonction de la nature et de la concentration en échantillon testé.

### RESULTATS

### Identification des résidus contributeurs du paratope de l'anticorps à la liaison CD4.

Dix-sept hexapeptides des séquences préalablement identifiées des régions variables de l'anticorps 13B8.2 [Bès, 2001b], et des séries d'analogues-alanine ont été synthétisés sur des membranes de cellulose par la méthode Spot pour identifier précisément les résidus critiques de l'anticorps impliqués dans la liaison au CD4.

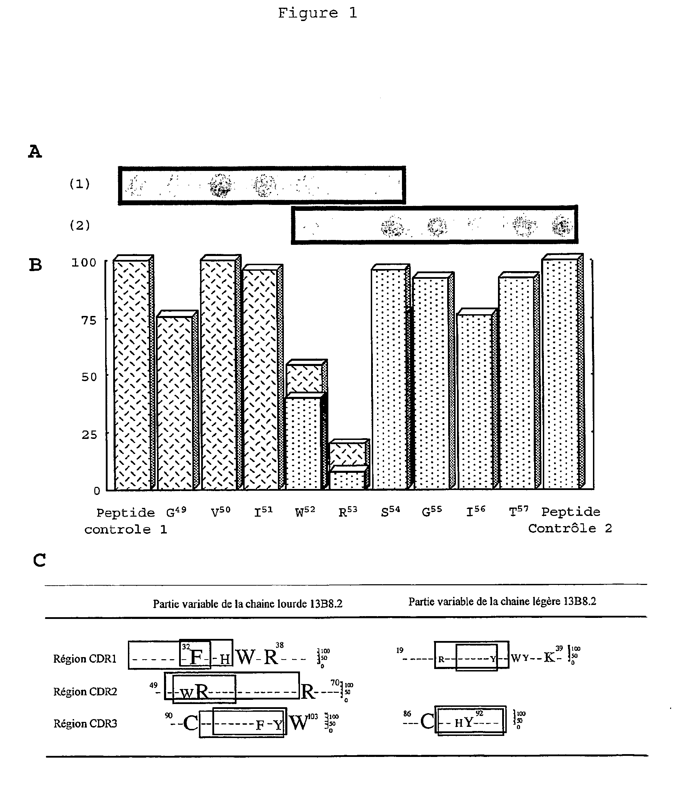

Une étude détaillée de la séquence 49-57 de la région CDR-H2 de l'anticorps 13B8.2 est montré Figures 1A et 1B en annexe.

La substitution du Trp⁵² du peptide ⁴⁹GVIWRS⁵⁴ (défini comme peptide contrôle 1) par un résidu alanine conduit à une diminution de 50% dans la capacité de liaison au CD4, alors que le changement de Arg⁵³ conduit à une perte totale de la réactivité pour l'antigène.

Les quatre autre remplacements des résidus du peptide ⁴⁹GVIWRS⁵⁴ par une alanine ne modifient pas leur capacité à se lier à l'antigène.

La contribution du Trp⁵² et du Arg⁵³ à la liaison avec la molécule CD4 a été confirmée par la méthode de balayage d'alanine du peptide ⁵²WRSGIT⁵⁷ (Figure 1A et 1B).

Des expériences similaires en utilisant des hexapetides ⁶¹VPFMSR⁶⁶, ⁶⁵SRLSIT⁷⁰ de la région CDR-H2 et de ses six alanine analogues permettent l'identification du résidu Arg⁶⁶ en tant que résidu de liaison de la molécule CD4 (Données non montrées).

Pris ensemble, il a été détermine que le motif contributeur pour la région H2 est ⁵²WR------------R⁶⁶ (Figure 1C) avec deux résidus Trp⁵² et Arg⁵³ appartenant au CDR.

De la même manière, le motif contributeur ³²F-HW-R³⁸ a été déterminé par analyse Spot alascan de trois hexapeptides ³¹TFGVHW³⁶, ³⁴VHWVRQ³⁹ et ³⁶WVRQSP⁴¹ de la région H1 du paratope de l'anticorps 13B8.2 ; Un des deux résidus (Phe³², His³⁵) selon la nomenclature de Kabat des IGMT (Figure 1C) est impliqué dans le CDR.

Pour les régions H3, le motif se liant à la molécule CD4 ⁹²C--------F-YW¹⁰³ comprenant les résidus Phe^{100K} et Tyr¹⁰² du CDR et deux autres résidus de structure (framework) ont été déterminés des trois hexapeptides ⁹⁰YYCAKN⁹⁵, ⁹²CAKNDP⁹⁷ et ⁹⁹TGFAYW¹⁰³.

L'étude des quatre hexapeptides ¹⁹VTFTCR²⁴, ²¹FTCRAS²⁶, ³²YLAWYQ³⁷ et ³⁵WYQQKQ⁴⁰ de la région L1 a identifié les résidus Arg²⁴, Tyr³², Trp³⁵, Tyr³⁶ et Lys³⁹ comme ceux contribuant à la liaison de la :molécule CD4.

Etant donné qu'il n'y a pas eu de réactivité observée dans la région L2 [Bès, 2001b], aucun motif de liaison n'a été identifié.

Le motif ⁸⁸C--HY⁹² contribue à la liaison par la molécule CD4, selon les résultats d'analyse par alascan SPOT des trois hexapeptides ⁸⁵TYYCQH⁹¹, ⁸⁸CQHHYG⁹³ et ⁹¹HYGNPP⁹⁶ de la région L3, avec les résidus His⁹¹ et Tyr⁹² appartenant au CDR (Figure 1C) .

Pris dans son ensemble dix-neuf résidus du paratope de l'anticorps 13B8.2 ont été initialement identifiés comme des résidus SCR se liant à la molécule CD4 par analyse alascan SPOT.

Toutefois, parmi eux, le résidu Arg⁶⁶ de la chaîne lourde et les résidus Arg²⁴ et Lys³⁹ de la chaîne légère ont été trouvés systématiquement être faiblement accessibles au solvant dans la structure tridimensionnelle des anticorps et n'ont jamais été identifiés préalablement comme étant des résidus critique dans la liaison de l'antigène [McCallun, 1996, http://www.rubic.rdg.ac.uk; Honneger, 2001, http://www.biochem.unizh.ch]

Par conséquent, seulement seize SCR du paratope de l'anticorps 13B8.2 ont été choisis pour la mutagenèse dirigée effectuée par la suite.

### Caractérisation des Fabs mutants, d'une alanine unique, de l'anticorps 13B8.2.

Les gènes des régions variables des chaînes lourdes et légères de l'anticorps 13B8.2 ont été utilisés comme matrices pour une mutagenèse dirigée par PCR chevauchant, pour remplacer chacun des SCR sélectionnés par un résidu alanine dans le contexte global du Fab de l'anticorps 13B8.2.

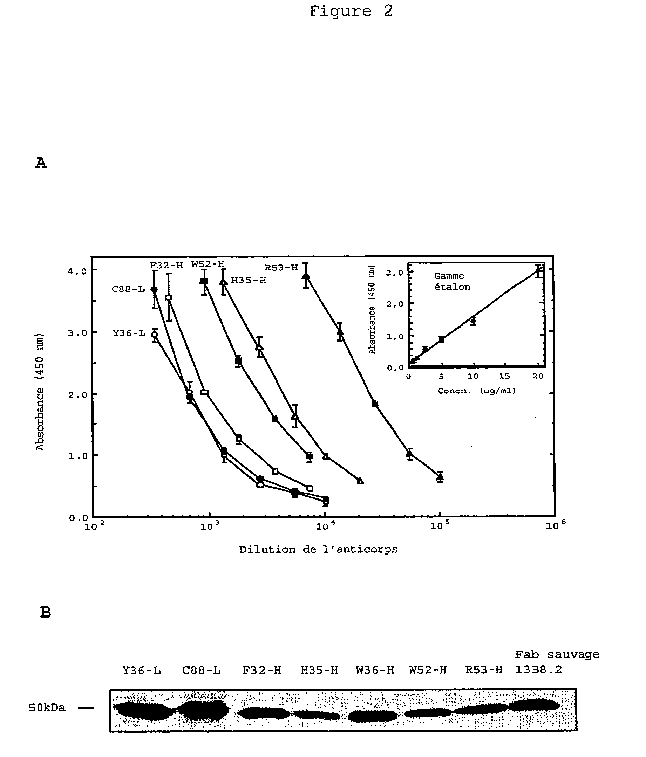

Après le clonage dans des vecteurs pBHuFd_₁ ou pBHuC_ appropriés, l'expression dans un système baculovirus/cellule d'insecte, la purification au moyen de protéine G, chacun des Fab mutants, ci-après désignés F32-H, H35-H, W36-H, R38-H, W52-H, R53-H, C92-H, F100K-H, Y102-H et W103-H pour ceux présentant des mutations sur le domaine variable de la chaîne lourde et Y32-L, W35-L, Y36-L, C88-L, H91-L et Y92-L pour ceux présentant des mutations sur le domaine variable de la chaîne légère) ont été de plus détectés et quantifiés par ELISA et Western blot pour la production d'immunoglobulines (Figures 2A et 2B).

Les séquences des régions des domaines VH et VL de chacun des Fabs mutants, par rapport à la séquence correspondante du Fab sauvage de l'anticorps 13B8.2 sont présentées dans le tableau III (Domaine VH) et le tableau IV (Domaine VL) ci-dessous.

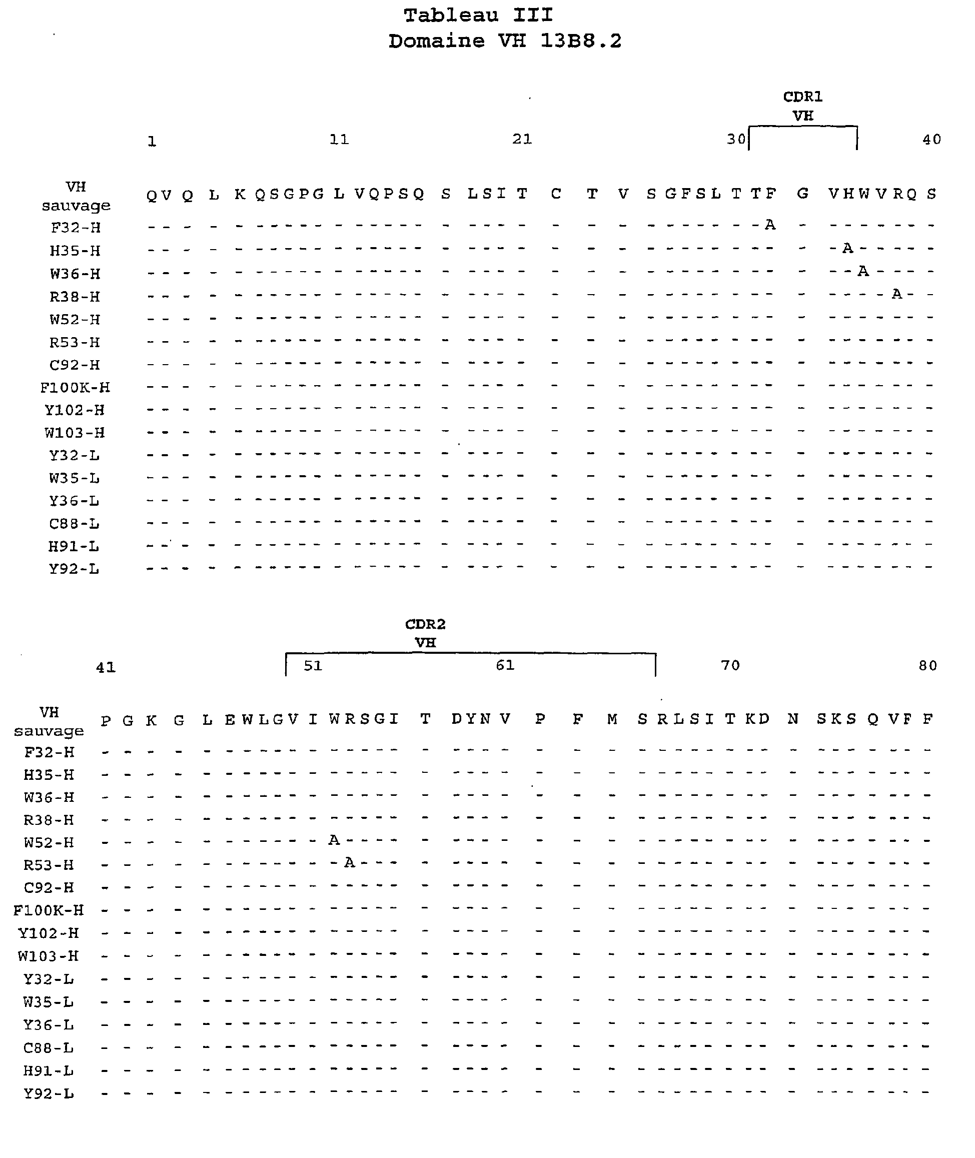

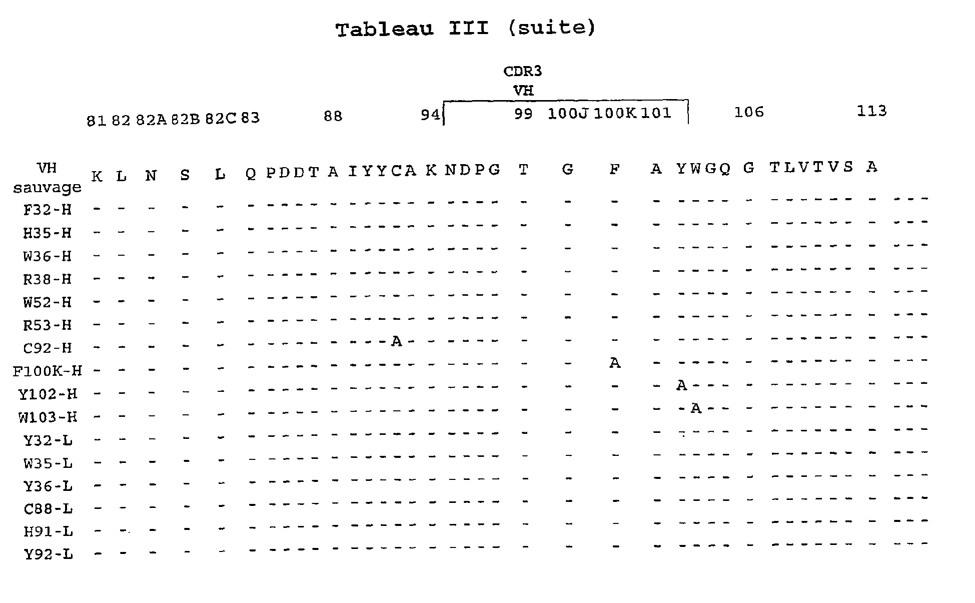

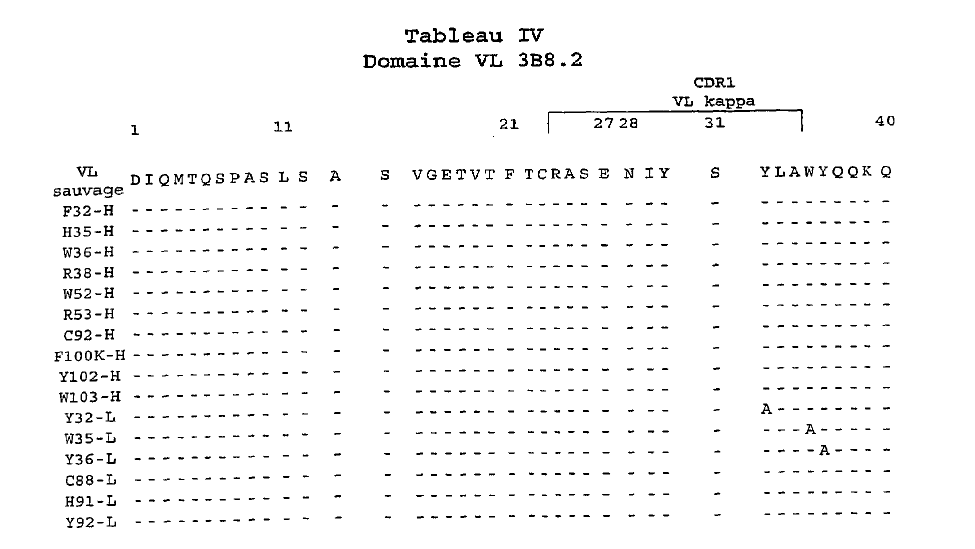

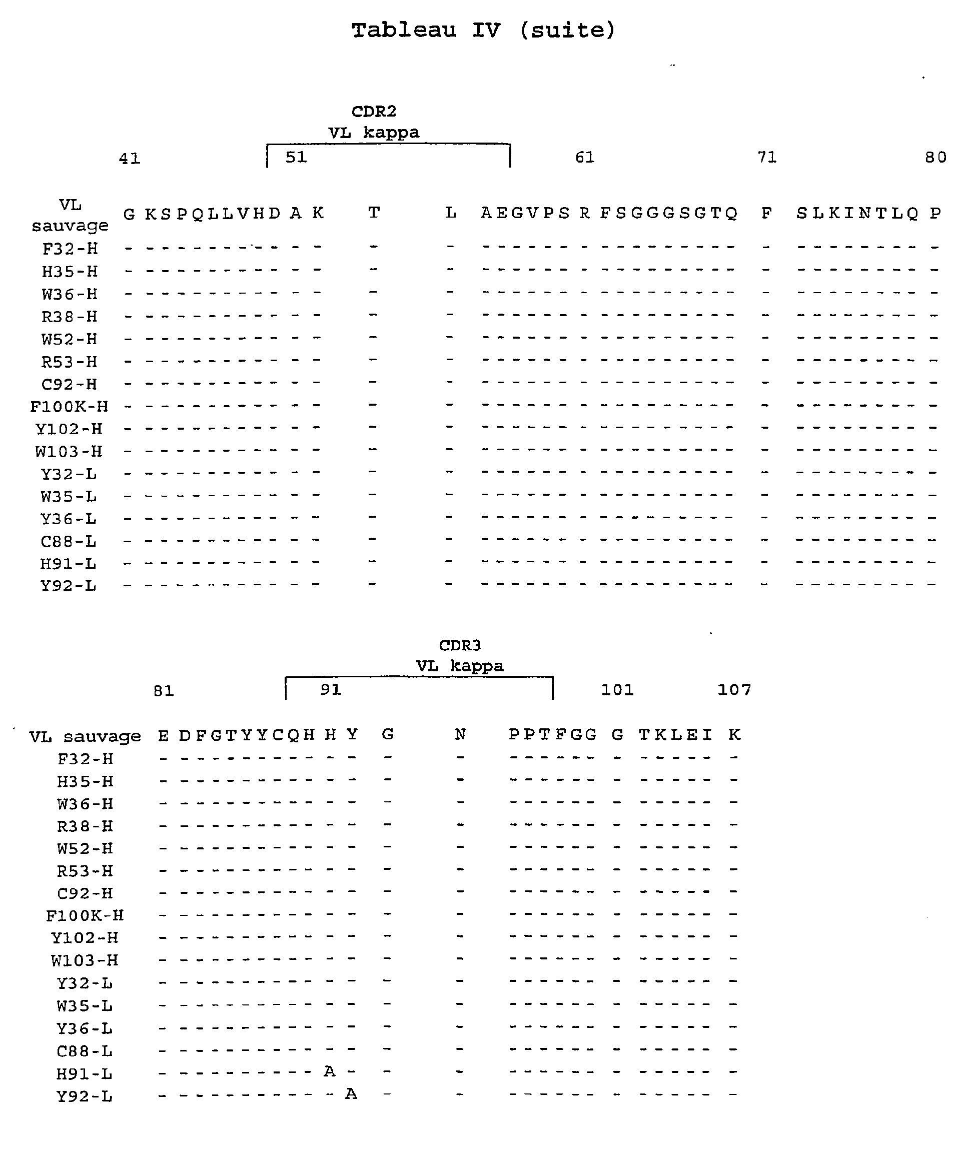

Comme le montrent les exemples pour les mutants Y36-L, C88-L, F32-H, H35-H, W52-H et R53-H, la production d'immunoglobuline, mise en évidence par un anticorps de capture dirigé contre la chaîne lourde et révélé par un anticorps dirigé contre la chaîne kappa conjuguée à la peroxydase, a été démontrée pour chaque Fab recombinant purifié.

Des analyses electrophorétiques sur gel d'acrylamide en présence de SDS (PAGE-SDS) par coloration au Bleu de Coommassie effectuée avec 1 µg de protéine chargée pour chaque Fab recombinant révèlent une bande unique à 50 Kdalton (données non-montrés), correspondant à la taille attendue d'un Fab correctement recombiné sous des conditions non-réductrices ; l'identité de la bande à 50 KD a ensuite été confirmée par Western blot, en utilisant un anticorps dirigé contre la chaîne kappa humaine (Figure 2B).

### Capacité de liaison de la molécule CD4 par les Fab mutants versus le Fab sauvage de l'anticorps 13B8.2.

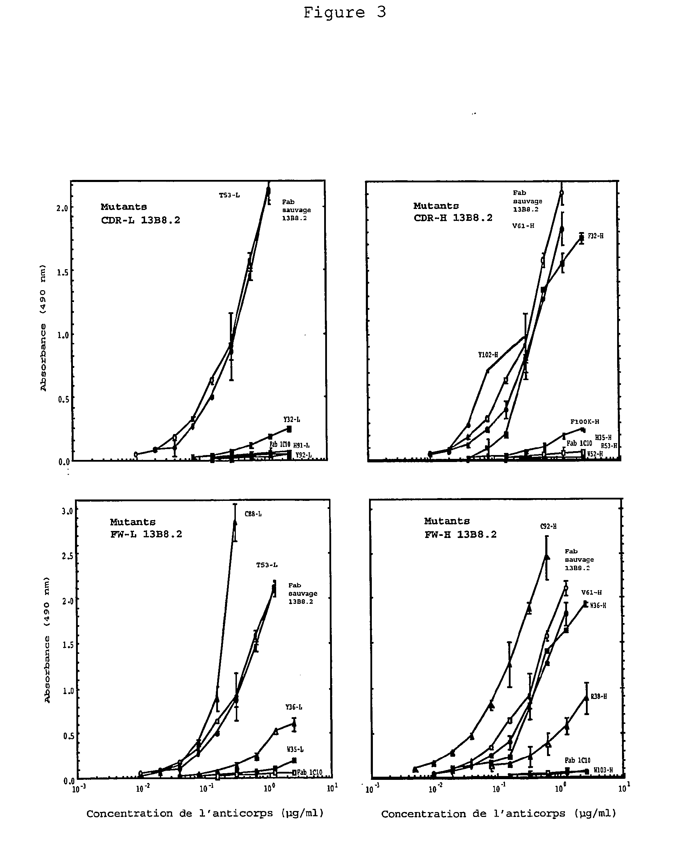

La capacité des Fab mutants versus le Fab sauvage pour lier la molécule de CD4 soluble a d'abord été vérifiée par une méthode ELISA (Figure 3) et ensuite quantifié par analyse BIAcore (Tableau II ci-dessous).

**TABLEAU II**

| *Determination au BIACORE de la cinétique d'interaction entre le CD4 fixé sur l e et les anticorps 13B8.2* | | | | |
|---|---|---|---|---|
| Anticorps | | *k ₐ* | *k _{d}* | *K _{D}* |
| | | *10 ⁴ S ⁻¹ M ⁻¹* | *10 ⁻⁴ S ⁻¹* | *nM* |
| Fab sauvage 13B8.2 | Exp. 1 | 0.38 | 1.08 | 28.4 |
| | Exp. 2 | 0.48 | 1.61 | 33.5 |
| | Exp. 3 | 0.25 | 0.64 | 25.6 |
| Fab contrôle 1C10 | | NM | NM | NM |
| Fab mutants 13B8.2 | | | | |
| Y32-L | | 0.00655 | 5.85 | 8931.2 |
| W35-L | Exp.1 1 | 4.94 | 9.23 | 18.7 |
| | Exp. 2 | 6.60 | 8.41 | 17.0 |
| Y36-L | Exp.1 | 2.56 | 6.51 | 25.4 |
| | Exp.2 | 0.50 | 5.70 | 11.4 |
| T53-L contrôle | | 2.21 | 3.65 | 16.5 |
| C88-L | | 3.80 | 4.09 | 10.8 |
| H91-L | | 0.0239 | 40.40 | 16903.7 |
| Y92-L | Exp.1 | 7.47 | 12.70 | 17.0 |
| | Exp. 2 | 7.07 | 8.89 | 12.5 |
| F32-H | Exp.1 | 1.83 | 2.48 | 13.5 |
| | Exp. 2 | 1.44 | 2.46 | 17.0 |
| | Exp. 3 | 1.48 | 2.50 | 16.9 |
| H35-H | | 0.0301 | 18.90 | 6279.0 |
| W36-H | | 1.76 | 2.71 | 15.4 |
| R38-H | | 0.0163 | 1.40 | 858.8 |
| W52-H | | 0.0237 | 8.59 | 3624.4 |
| R53-H | | 0.0359 | 105.00 | 29247.9 |
| V61-H contrôle | Exp. 1 | 8.68 | 1.72 | 2.0 |
| | Exp. 2 | 11.20 | 3.24 | 2.9 |
| C92-H | | 9.61 | 3.58 | 3.7 |
| F100K-H | | 0.0562 | 4.75 | 845.1 |
| Y102-H | | nd*^{b}* | nd | nc*^{c}* |
| W103-H | | 0.0278 | 11.2 | 4028.7 |

| | | | | |
|---|---|---|---|---|
| *^{a}* Non *^{b}* Non '' *^{c}* Non calculé | | | | |

En ELISA, l'activité de liaison au CD4 du Fab sauvage a été démontrée dans la gamme allant de 19,5 à 1250 ng/ml (Figure 2) alors qu'aucune liaison n'a été observée avec le Fab recombinant 1C10 anti-digoxine.

Des réactivités dose-dépendantes pour le CD4 similaires à celles observées pour le Fab sauvage ont été mises en évidence pour les Fab mutants contrôles T53-L et V61-H, ainsi que pour les Fabs mutants sur les positions caractérisées en Spot, C88-L, F32-H, W36-H, C92-H et Y102-H.

D'autre part, la mutation d'une alanine au niveau des résidus caractérisés en Spot Tyr³², His⁹¹ et Tyr⁹² des régions CDR-L, His³⁵, Trp⁵², Arg⁵³ et Phe^{100K} des régions CDR-H, Trp³⁵ et Tyr³⁶ des régions FWs de la chaîne légère, et Arg³⁸ et Trp¹⁰³ des régions FWs de la chaîne lourde affectent la liaison à la molécule CD4 de manière dose-dépendant (Figure 3).

Ces résultats ont été confirmés au moyen de la technologie BIAcore étant donné que la plupart des Fabs mutants présentant une perte de leur capacité de liaison de la molécule CD4 par ELISA, tels que les Fabs Y32-L, H91-L, H35-H, R38-H, W52-H, R53-H, F100K-H et W103-H, ont montré une diminution du K_{D} de l'ordre de 100 à 1000 fois, alors que ceux qui montrent le maintien de leur capacité de liaison à la molécule CD4 en ELISA, ont montré des valeurs de K_{D} similaires à celles calculées pour le Fab sauvage de l'anticorps 13B8.2 (Table I). Finalement, les Fabs mutants W35-L, Y36-L et Y92-L, présentant une diminution de leur capacité de liaison à la molécule CD4 en ELISA, ont montré des valeurs de K_{D} similaires à celles du Fab sauvage de l'anticorps 13B8.2, mais avec une vitesse de dissociation plus élevée.

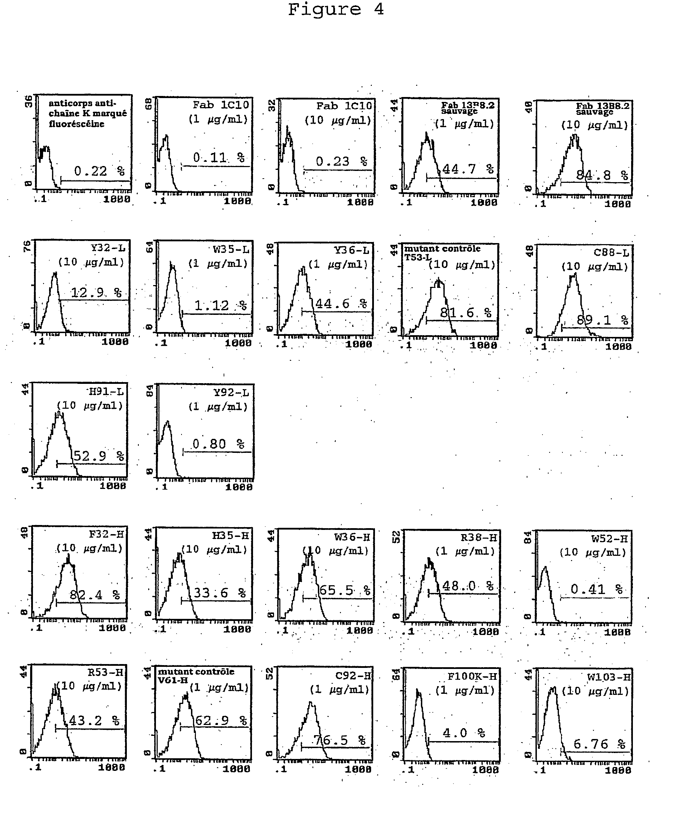

Pour déterminer la liaison des fragments au CD4 membranaire, des cellules T AN2.01 CD4+ ont été analysées par marquage immunofluorescent indirect et cytométrie de flux (Figure 4) .

Une forte coloration des cellules T est obtenue avec le Fab sauvage de l'anticorps 13B8.2 d'une manière dose-dépendante, alors que les contrôles avec le Fab 1C10 anti-digoxine ou l'anticorps anti-chaîne kappa marqué à la fluorescéine ne se lient pas auxdites cellules.

Des expériences similaires en utilisant des cellules T AN2.01 CD4 négatives ont montré qu'il n'y a pas de liaison non spécifique, ni avec le Fab sauvage, ni avec les Fab ayant leurs alanines mutantes correspondantes.

D'autre part, une défaillance dans la liaison à la molécule CD4 desdites cellules a été observée pour les mutants ayant un remplacement d'alanine sur les résidus Tyr³², Trp³⁵, His⁹¹ et Tyr⁹² de la chaîne légère, et His³⁵, Trp⁵², Arg⁵³, Phe^{100K} et Trp¹⁰³ de la chaîne lourde.

Activités biologiques des Fabs ayant des résidus mutés en alanine par rapport au Fab sauvage de l'anticorps 13B8.2 dans les réponses impliquant la molécule CD4.

Des cellules présentatrices d'antigène (APC) EBV-Lu stimulées avec le peptide Pep24 co-cultivées avec des cellules T répondantes pdb10F conduisent à la sécrétion lymphocytaire d'Il2 à la suite de la présentation de l'antigène.

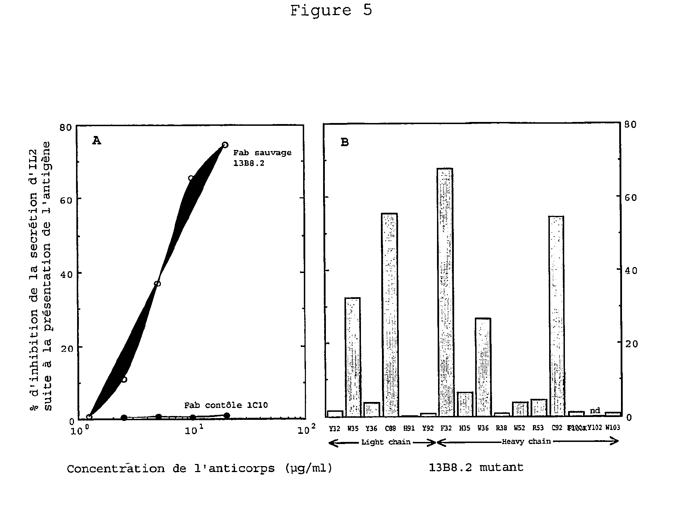

Comme illustré Figure 5, une inhibition de la sécrétion d'Il2 dose-dépendante est démontrée à la suite de l'incubation du Fab anti-CD4 sauvage de l'anticorps 13B8.2 dans ce modèle d'activation cellulaire T.

Aux mêmes concentrations, le contrôle Fab 1C10 ne montre aucune activité inhibitrice.

Les Fab mutants montrant une capacité de liaison à la molécule CD4 similaire à celle du Fab sauvage, tels que C88-L, F32-H, W36-H et C92-H, maintiennent aussi leurs propriétés inhibitrices sur la présentation d'antigène.

Par contre les autres mutations au niveau des SCR abrogent totalement ou partiellement l'inhibition de la fonction de présentation de l'antigène des Fabs anti-CD4 mutants (Figure 5).

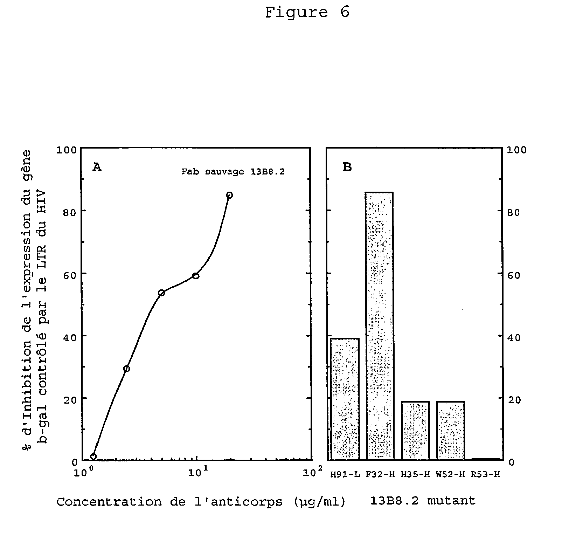

Ces résultats sont corrélés avec ceux obtenus par l'étude des propriétés inhibitrices des Fab mutants H91-L, F32-H, H35-H, W52-H et R53-H versus le Fab sauvage dans l'activité promoteur du VIH-1 (Fig. 6). Dans ce cas, les Fabs mutants montrant une défaillance dans leur capacité de liaison de la molécule CD4 (H91-L, H35-H, W53-H et R53-H), ne sont pas capables de bloquer l'expression du gène reporteur de β-galactosidase sous contrôle du LTR du VIH-1, alors que le Fab mutant F32, avec une capacité de liaison de la molécule CD4 préservée comme le Fab sauvage, inhibe l'expression du gène reporteur.

Considérés ensemble, les mutations en alanine, préalablement identifiées en Spot conduisent à une défaillance de la liaison à la molécule CD4 corrélée avec un défaut dans les activités biologiques des Fabs mutants.

Analyse structurelle de la liaison des Fabs mutants à la molécule CD4 à partir d'un modèle informatique des régions variables de l'anticorps 13B8.2.

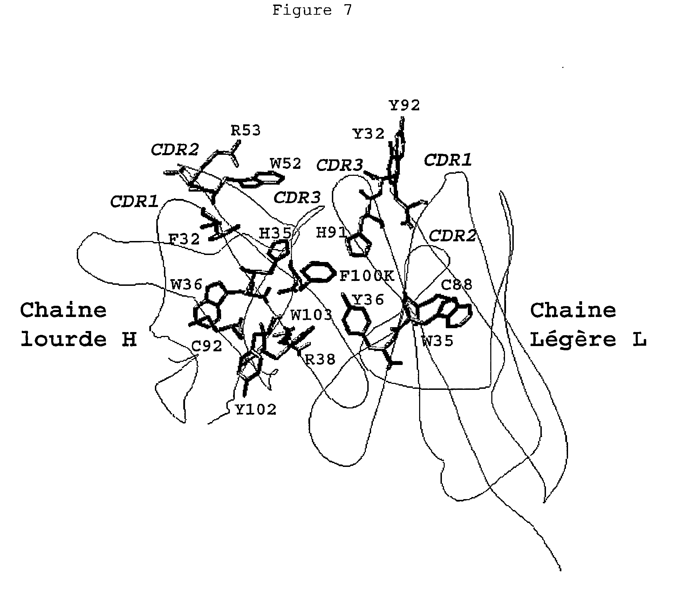

Un modèle tridimensionnel du paratope de l'anticorps 13B8.2 a été obtenu en effectuant l'alignement des acides aminés de la séquence de l'anticorps monoclonal 13B8.2 avec la bibliothèque de séquences AbM, selon la définition de structures (« frameworks ») de classes canoniques dans AbM et une technique de recherche conformationnelle en utilisant CONGEN pour le CDR H3 (Figure 7).

La structure des anticorps 1fdl et 1nld a fourni la matrice pour les régions FWs des chaînes légères et lourdes de l'anticorps 13B8.2 respectivement.

Les CDRs , à l'exception du CDR-H3, ont été construits sur la base des classes canoniques.

Les boucles connues pour avoir les séquences les plus homologues de la même classe canonique ont été utilisées.

Les CDR-L1, CDR-L2 et CDR-L3 ont été construits en utilisant la structure tridimensionnelle de l'anticorps lfdl et les CDR-H1 et CDR-H2 ont été construits en utilisant la structure 1nld.

La boucle flexible CDR-H3 n'as pas été décrite comme une classe canonique et a été construite en utilisant une recherche dans les bases de données combinée avec une recherche conformationnelle (CONGEN).

La structure, comme attendu, présente 2 ponts disulfure entre les Cys22 et 92 et le feuillet béta de la chaîne lourde et les résidus Cys23 et 88 du feuillet béta de la chaîne légère.

L'analyse de l'accessibilité au solvant des acides aminés de l'anticorps 13B8.2 et de l'orientation des chaînes latérales des résidus critiques montre que la plupart des résidus SCR liant la molécule CD4 confirmés par mutagènes dirigées sont orientés structurellement à l'intérieur du site de combinaison avec CD4.

D'autre part, les Fabs mutants au niveau des résidus Phe³², Trp³⁶, Cys⁹², Tyr¹⁰² et Cys⁸⁸ , dont les mutations n'altèrent pas la liaison au CD4 dans le contexte du Fab entier, montrent que leurs chaînes latérales ne sont pas accessibles au solvant (Cys⁹², Trp³⁶, Cys⁸⁸) ou pointent dans une direction opposée celle de l'orientation principale de la poche de liaison de CD4, comme pour Phe³² et Tyr¹⁰² ; confirmant ainsi structurellement que leur contribution à la liaison au CD4 est peu probable.

Plus spécifiquement, les deux cystéines ne sont pas accessibles au solvant et sont impliquées dans des ponts disulfure, qui diminuent leur probabilité d'interagir avec CD4.

La plupart de résidus de contact SCR confirmés par mutagenèse dirigée sont des résidus aromatiques et/ou des résidus chargés.

A l'entrée d'un site de liaison avec l'antigène correspondant à une superficie de 150 Amsgströms carrés, les résidus Trp⁵² et Arg⁵³ de la boucle CDR-H2 et les résidus Tyr³² et Tyr⁹² des boucles CDR-L1 et CDR-L3 respectivement, contribuent de chaque coté à la liaison avec CD4. Le résidu Tyr³⁰ de la boucle CDR-L1 est aussi exposé au solvant et constitue un patch hydrophobe avec les résidus Tyr³² et Tyr^{92.}

Le fond du site de liaison à CD4, couvrant une superficie de 75 Angströms carrés est constitué par un cluster de résidus aromatiques, principalement chargés positivement, définis comme les résidus His³⁵, Phe^{100K} des boucles CDR-H1 et des boucles CDR-H3 respectivement et His⁹¹ de la région CDR-L3.

Quatre résidu additionnels (Arg³⁸, Trp¹⁰³ de la chaîne lourde et Trp³⁵ et Tyr³⁶ de la chaîne légère), avec une faible accessibilité au solvant et appartenant essentiellement au FW, stabilisent probablement les conformations de squelette des régions impliquées dans la poche de liaison de CD4 ou façonnent l'interface H/L de l'anticorps.

La présence de trois résidus chargés positivement sous-jacents à l'entrée (Arg⁵³ du CDR-H2) et le centre du site de liaison de CD4 (His³⁵ du CDR-H1 et His⁹¹ du CDR-L3) suggèrent que des interactions électrostatiques peuvent constituer un des éléments majeurs de la liaison entre l'anticorps 13B8.2 et son épitope sur la région homologue du CDR3 sur la molécule CD4.

L'analyse par modélisation moléculaire de la région CDR-L2 montre que la boucle L2 est relativement bien exposée, expliquant la raison pour laquelle l'activité de non- liaison du CD4 a été préalablement démontrée par la méthode Spot [Bès, 2001b].

### Discussion

La définition de résidu critique impliqué dans la liaison avec l'antigène d'un paratope d'un anticorps donné est une condition requise pour le guidage de la construction de variantes avec des activités améliorées.

La cristallographique de rayons X parfois combinée avec la mutagenèse dirigée et/ou la modélisation moléculaire est une méthode de choix pour la conception de sites de combinaison antigène/anticorps.

De telles analyses du paratope structurel (avec des coordonnées atomiques) sont cependant limitées à certains complexes, dépendant de la disponibilité de l'antigène et de l'anticorps (des quantités importantes sont souvent requises)., le niveau de modifications post transductionnelles de l'antigène et la qualité de la cristallogenèse du complexe, celles-ci sont particulièrement cruciales pour les protéines de grande taille.

En février 2002, les coordonnées atomiques des complexes antigène/anticorps d'environ seules vingt protéines différentes ont été décrites dans la banque Protein Data Bank.

La Demanderesse a maintenant prouvé que la méthode Spot en parallèle avec la synthèse peptidique constitue une approche complémentaire pour la cartographie fine des résidus critiques du paratope d'un anticorps impliqué dans la liaison avec l'antigène, exempts des limitations précédemment citées.

Cette technique de cartographie présente un intérêt particulier pour les protéines qui ne sont pas disponibles en grande quantité. Pour mener à bien de telles expériences, il suffit de disposer des séquences d'acide aminé des régions variables d'un anticorps, des faibles quantités d'antigène et de la technique de synthèse peptidique Spot

A partir de l'anticorps anti-CD4 13B8.2, environ 70% des résidus se liant à la molécule CD4, selon les analyses effectuées par la méthode Spot ont été confirmés par mutagenèse dirigée. Ces résultats sont corrélés avec les arguments indirects préalables indiquant qu'un certain nombre de résidus choisis via des mutations somatiques d'un anticorps anti-troponine étaient des résidus contributeurs dans le format Spot [Laune, 2002] et que 65% des résidus SCR identifiés d'un anticorps anti-lysozyme [Laune, 1998], étaient corrélés avec des résidus contributeurs préalablement définis par cristallographique de rayons X du complexe lysozyme/HyHel-5 [Scheriff, 1987; Cohen, 1996].

De plus, dix parmi onze résidus SCR impliqués dans la liaison à la molécule CD4 (Tyr³², Trp³⁵, Tyr³⁶, His⁹¹ et Tyr⁹² de la chaîne légère de l'anticorps 13B8.2; His³⁵, Trp⁵², Arg⁵³, Phe^{100K} et Trp¹⁰³ de la chaîne lourde de l'anticorps 13B8.2) sont localisés dans des positions déjà identifiées comme étant des sites de contact avec la molécule CD4 [McCallum, 1996 ; Honneger, 2001],, alors que quatre résidus sur cinq, exclus du paratope du CD4 par mutagenèse dirigée n'appartiennent pas à cette classe de « contact ».

De manière intéressante, bien que localisé dans une position déjà définie comme étant en contact avec l'antigène [McCallum, 1996; Honneger, 2001], le dernier résidu Phe³² de la chaîne lourde de l'anticorps 13B8.2n'a jamais été repéré comme un résidu acide aminé critique dans cette position [Honneger, 2001].

Comme il a été suggéré [Laune, 2002], ces résidus faux positifs en Spot peuvent également être expliqués du fait que le format du peptide expose des résidus de l'anticorps normalement masqués dans le paratope fonctionnel.

Ceci est souligné par l'étude de modélisation de l'anticorps 13B8.2, dans laquelle seuls les vrais résidus SCR liant la molécule CD4 sont structurellement orientés à l'intérieur de la poche liant l'antigène, en accord avec la vision générale de que la position et l'orientation d'un résidu par rapport au centre du site de combinaison sont des points clé pour leur aptitude à lier l'antigène [McCallum, 1996] .

Parmi les résidus critiques, quatre résidus sont moins accessibles dans le site de combinaison avec l'antigène, suggérant qu'ils n'interagissent pas structurellement avec la molécule CD4, mais sont influencés indirectement par le paratope CD4.

De fait, les résidus Trp³⁵, Tyr³⁶ de la chaîne légère et Trp¹⁰³ de la chaîne lourde, sont localisés dans des positions appartenant à la zone Vernier [Foote, 1992], qui contient des résidus qui ajustent la structure CDR et affinent l'adaptation à l'antigène.

De plus, des acides aminés en position 36 de la chaîne légère et en position 103 de la chaîne lourde montrent une réduction de l'accessibilité de leurs chaînes latérales après la formation d'interfaces dimères entre les régions variables des chaînes lourdes et légères [Honneger, 2001] , suggérant qu'ils sont importants pour déterminer la forme de la poche de liaison à l'antigène, tel que l'interphase H/L .

D'autre part, les résidus SCR liant CD4 qui interagissent probablement directement avec l'antigène, tels que His³⁵, Trp⁵², Arg⁵³ et Phe^{100K} de la chaîne lourde de l'anticorps 13B8.2 et Tyr³², His⁹¹ et Tyr⁹² de la chaîne légère de l'anticorps 13B8.2 , sont localisés dans des positions critiques pour le contact protéine-antigène, identifiés préalablement comme des étant bien accessibles au solvant et comme ayant une réduction importante de la surface de leur chaîne latérale accessible après la liaison avec l'antigène [Honneger, 2001].

Moins de 1% des toutes les séquences des régions variables ont été décrites comme des séquences manquant d'un résidu cystéine en position L88 ou H92.

Avec le résidu Cys²³ de la chaîne légère et le résidu Cys²² de la chaîne lourde respectivement, ils forment des ponts disulfure entre les feuillets béta de manière à maintenir la stabilité thermodynamique et le pliement de l'anticorps.

Alors que la plupart d'anticorps recombinant exprimés dans des bactéries d'où la cystéine est absente montrent une défaillance dans leurs propriétés de liaison à l'antigène, des tels anticorps entiers fonctionnels ont été décrits chez les eucaryotes [Vrana, 1976].

Les observations de la Demanderesse concernant les Fab mutants C88-L et C92H de l'anticorps 13B8.2 exprimés dans un système baculovirus/cellule d'insecte reconnaissent encore le CD4 et maintiennent également leurs propriétés biologiques renforce l'hypothèse qu'une voie alternative telle que la dérivation au moyen d'une glutathione de la cystéine résiduelle peut se produire dans un système d'expression eucaryote, tel que celui décrit pour l'expression du lysozyme mutant Cys-défectif chez la levure [Taniyama, 1990] et comme suggéré pour l'expression d'anticorps entier Cys-défectifs [Proba, 1997], conduisant à la production de protéines fonctionnelles manquant de cystéine.

Probablement d'autres résidus, essentiellement localisés dans les régions CDR-H3 et CDR-L3 et non identifiés par la méthode Spot peuvent contribuer à la liaison à la molécule CD4.

Comme suggéré par la modélisation moléculaire assistée par ordinateur de l'anticorps 13B8.2, les résidus Asn⁹⁵ et Thr⁹⁹ de la chaîne lourde, et Asn⁹⁴ de la chaîne légère montrent une orientation de leurs chaînes latérales vers le centre du site de recombinaison avec Cd4 (données non-montrées).

De plus, ils sont localisés dans une position d'acides aminés déjà définie comme étant une position de contact de l'antigène et la protéine [Honneger, 2001].

Des expériences préliminaires en utilisant le mutant N95-H de l'anticorps 13B8.2 argumentent en faveur d'une telle contribution.

Pris dans leur ensemble ces observations soulignent le besoin de combiner la modélisation moléculaire des régions variables d'un anticorps donné avec des analyses Spot alascan de peptides de 6 ou 12 acides aminés du paratope de manière à définir plus précisément les résidus critiques pour la liaison avec l'antigène.

Bien que des complexes de cristallisation du complexe CD4 anticorps n'aient pas été décrits, la modélisation moléculaire de l'anticorps OKT4A, qui reconnaît un épitope lié à la boucle homologue du domaine CDR2-*like* du domaine 1 de la molécule CD4, a permis l'étude du site de combinaison de cet anticorps [Pulito, 1996]. De manière intéressante, des caractéristiques similaires dans la conformation globale peuvent être notées entre la poche de liaison de l'anticorps OKT4A et l'anticorps 13B8.2.

Deux résidus chargés (Lys⁹⁵ et Asp^{100A}) de la chaîne lourde de l'anticorps OKT4A centrent le site de liaison comme c'est le pour les résidus His³⁵ et His⁹¹ des chaînes lourdes et légères respectivement de l'anticorps 13B8.2.

Le rôle de tels résidus chargés positivement de l'anticorps 13B8.2 peut être souligné car (i) la liaison au CD4 est augmentée par l'incubation du Fab de l'anticorps 13B8.2 à pH 6 .0 auquel 50% des résidus histidine sont chargés positivement par rapport à seulement 7% à pH 7,2 (données non-montrées) et (ii) l'épitope homologue du CDR3 de l'anticorps 13B8.2 implique essentiellement des résidus Glu⁸⁷ et Asp⁸⁸ chargés négativement, de la molécule CD4. [Sattentau, 1989] suggérant que des interactions électrostatiques fortes sont de grande importance dans le site de combinaison 13B8.2/CD4.

Sous-jacents à ces résidus, un cluster de résidus ayant des chaînes latérales aromatiques aussi bien pour l'anticorps 13B8.2 que pour l'anticorps OKT4A contribuent pour l'essentiel à la liaison.

Le fond de la poche de liaison à CD4 implique d'autres résidus masqués, appartenant essentiellement aux régions cadre (frameworks), tels que Trp³⁵, Tyr³⁶, Arg³⁸ et Trp¹⁰³ de l'anticorps 13B8.2, et Ala³⁴, Leu⁸⁹, Ser³⁵ et Ala⁵⁰ pour l'anticorps OKT4A qui peuvent être critiques pour une conformation appropriée du site de combinaison [Pulito, 1996].

L'anticorps 13B8.2 montre une inhibition post-entrés de la transcription du VIH et de l'activation des cellules T [Pour une revue d'ensemble consulter Briant et al., 2000], des tels effets biologiques étant abrogés en utilisant des fragments Fab mutants qui dérèglent la liaison à la molécule CD4.

En d'autres termes, ces résultats indiquent qu'au moins les résidus His³⁵, Arg³⁸, Trp⁵², Arg⁵³, Phe^{100k} et Trp¹⁰³ de la chaîne lourde de l'anticorps 13B8.2 et les résidus Tyr³², Trp³⁵, Tyr³⁶, His⁹¹ et Tyr⁹² de la chaîne légère de l'anticorps 13B8.2 sont particulièrement critiques pour maintenir les effets biologiques souhaités de l'anticorps 13B8.

### REFERENCES BIBLIOGRAPHIQUES

Briant, L., and Devaux, C. (2000) Adv Pharmacol 48, 373-407
Gorman, S.D., Clark, M.R., Routledge, E.G., Cobbold, S.P., and Waldmann, H. (1991) Proc. Natl. Acad. Sci. USA 88, 4181-4185
Pulito, V.L., Roberts, V.A., Adair, J.R., Rothermel, A.L., Collins, A.M., Varga, S.S., Martocello, C., Bodmer, M., Jollife, L.K. and Zivin, R.A. (1996) J Immunol 156, 2840-2850
Reimann, K.A., Lin, W., Bixler, S., Browning, B., Ehrenfels, B., Lucci, J., Miatkowski, K., Olson, D., Parish, T.H., Rosa, M.D., Oleson, F.B., Hsu, Y.M., Padlan, E.A., Letvin, N.L., and Burkly, L.C. (1997), Aids Res. Hum. Retrovir. 13, 933-943
Fishwild, D.M., O'Donnell, S.L., Bengoechea, T., Hudson, D.V., Harding, F., Bernhard, S.L., Jones, D., Kay, R.M., Higgins, K.M., Schramm, S.R., and Lonberg, N. (1996) Nat Biotechnol 14, 845-851
Bès, C., Cerutti, M., Briant-Longuet, L., Bresson, D., Peraldi-Roux, S., Pugnière, M., Mani, J-C., Pau, B., Devaux, C., Granier, C., Devauchelle, G., and Chardès, T. (2001a) Hum. Antibodies 10, 67-76
Benkirane, M., Corbeau, P., Housset, V., and Devaux, C. (1993) Embo J. 12, 4909-4921
Benkirane M, Hirn M, Carriere D, Devaux C. J Virol. 1995 Nov;69(11):6898-903.
Dhiver, C., Olive, D., Rousseau, S., Tamalet, C., Lopez, M., Galindo, J.R., Mourens, M., Hirn, M., Gastaut, J.A., and Mawas, C. (1989) Aids 3, 835-842
Deckert, P.M., Ballmaier, M., Lang, S., Deicher, H., and Schedel, I. (1996) J Immunol 156, 826-833
Schedel, I., Sutor, G.C., Hunsmann, G., and Jurkiewicz, E. (1999) Vaccine 17, 1837-1845
Bès, C., Briant-Longuet, L., Cerutti, L., De Berardinis, P., Devauchelle, G., Devaux, C., Granier, C., and Chardès, T. (2001b) FEBS Lett. 508, 67-74
Frank, R. (1992) Tetrahedron 48, 9217-9232
Laune, D., Molina, F., Ferrières, G., Villard, S., Bès, C., Rieunier, F., Chardès, T., and Granier, C. (2002) J. Immunol. Methods in press
Laune, D., Molina, F., Ferrières, G., Mani, J-C., Cohen, P., Simon, D., Bernardi, T., Piechaczyk, M., Pau, B., and Granier, C. (1997) J. Biol. Chem. 272, 30937-30944.
Laune D, Pau B, Granier C.Clin Chem Lab Med. 1998 Jun; 36 (6) : 367-71.
Cohen, G.H., Sheriff, S., and Davies, D.R. (1996) Acta Crystallogr. Sec. D 52, 315-326.
Maddon, P.J., Molineaux, D.E., Maddon, K.A., Zimmerman, M., Godfrey, M., Alt, F.W., Chess, L., and Axel, R. (1987) Proc. Natl. Acad. Sci. USA 84, 9155-9159
Corbeau, P., Benkirane, M., Weil, R., David, C., Emiliani, S., Olive, D., Mawas, C., Serres, A., and Devaux, C. (1993) J. Immunol. 150, 290-301
Manca, F., De Berardinis, P., Fenoglio, D., Ombra, M.N., Li Pira, G., Saverino, D., Autiero, M., Lozzi, L., Bracci, L., and Guardiola, J. (1996) Eur J Immunol 26, 2461-2469
Briant, L., Robert-Hebmann, V., Acquaviva, C., Pelchen-Matthews, A., Marsh, M., and Devaux, C. (1998) J. Virol. 72, 6207-6214
Poulin, L., Evans, L.A., Tang, S., Barboza, A., Legg, H., Littman, D.R., and Levy, J.A. (1991) J. Virol. 65, 4893-4901
Monnet, C., Laune D., Laroche-Traineau J., Biard-Piechaczyk M., Briant L., Bès C., Pugniere M., Mani J. C., Pau B., Cerutti M., Devauchelle G., Devaux C., Granier C., and Chardès T. (1999) J. Biol. Chem. 274, 3789-3796
Chardès, T., Villard, S., Ferrieres, G., Piechaczyk, M., Cerutti, M., Devauchelle, G., and Pau , B. (1999) FEBS Lett 452, 386-394
Ho, S.N., Hunt, H.D., Horton, R., Pullen, J.K., and Pease, L. (1989) Gene 77, 51
Poul, M-A., Cerutti, M., Chaabihi, H., Devauchelle, G., Kaczorek, M., and Lefranc, M-P. (1995) Immunotechnology 1, 189-196
Karlsson, R., Roos, H., Fägerstam, L., and Persson, B. (1994) Methods (Orlando) 6, 99-110
Rees, A. R., Martin, A. C. R. , Pedersen, J.T., and Searle, S. M. J. (1992). ABM, a computer program for modeling variable regions of antibodies. Oxford Molecular Ltd, Oxford, UK
Foote, J., and Winter, G. (1992) J. Mol. Biol. 224, 487-499
Vrana, M., Tomasic, J., and Glaudemans, C.P.J. (1976) J. Immunol. 116, 1662-1664
Taniyama, Y., Seko, C., and Kikuchi, M. (1990) J. Biol. Chem. 265, 16767-16771
Proba, K., Honegger, A., and Plückthun, A. (1997) J. Mol. Biol. 265, 161-172
Sattentau, Q.J., Arthos, J., Deen, K., Hanna, H., Healey, D., Beverley, P.C., Sweet, R., and Truneh, A. (1989) J Exp Med 170, 1319-1334

## Revendications

1. Fragment Fab mutant de l'anticorps anti-CD4 13B8.2, **caractérisé en ce qu'**il lie la molécule CD4 et **en ce qu'**il comporte une mutation d'au moins un résidu F32, W36, C92, Y102 dans le domaine variable V_{H} de la chaine lourde et/ou C88 dans le domaine variable Vk de la chaîne légère.

2. Fragment Fab mutant selon la revendication 1, **caractérisé en ce qu'**il comporte une mutation C88L dans le domaine variable V_{κ}, ou une mutation F32H, W36H, C92H ou Y102H dans le domaine variable V_{H}.

3. Composition pharmaceutique comprenant à titre de principe actif un fragment Fab mutant selon l'une des revendications 1 ou 2, éventuellement en présence d'un excipient approprié, ledit fragment Fab mutant étant utilisé à une concentration comprise entre 0,01 mg/kg et 2 mg/kg, de préférence entre 0,1 et 0,4 mg/kg en poids du patient à traiter.

4. Utilisation d'une composition pharmaceutique selon la revendication 3, pour la préparation d'un médicament destiné à la prévention ou au traitement d'une pathologie autoimmune.

5. Utilisation selon la revendication 4, **caractérisée en ce que** la pathologie autoimmune est choisie parmi la polyarthrite rhumatoïde, le psoriasis ou le lupus érythémateux.

6. Utilisation d'une composition pharmaceutique selon la revendication 3, pour la préparation d'un médicament destiné à la prévention ou au traitement des réactions d'intolérance immunologique.

7. Utilisation selon la revendication 6, **caractérisée en ce que** la réaction d'intolérance immunologique est induite suite à une transplantation.

8. Utilisation d'une composition pharmaceutique selon la revendication 3, pour la préparation d'un médicament destiné à la prévention ou au traitement des réactions du type greffon vers l'hôte.

9. Utilisation d'une composition pharmaceutique selon la revendication 3, pour la préparation d'un médicament destiné à la prévention ou au traitement de cancers impliquant la molécule CD4.

10. Utilisation selon la revendication 9, **caractérisée en ce que** le cancer impliquant la molécule CD4 est choisi parmi un lymphome CD4+ ou un lymphome de Cesari.

11. Utilisation d'une composition pharmaceutique selon la revendication 3, pour la préparation d'un médicament destiné à la prévention ou au traitement d'immunodéficiences liées à une infection virale.

## Claims

1. Mutant Fab fragment of 13B8.2 anti-CD4 antibody, **characterized in that** it binds to the CD4 molecule and it comprises a mutation of at least one residue F32, W36, C92, Y102 in the variable domain V_{H} of the heavy chain and/or C88 in the variable domaine Vₖ of the light chain.

2. Mutant Fab fragment according to claim 1, **characterized in that** it comprises a mutation C88L in the variable domain Vₖ, or a mutation F32H, W36H, C92H or Y102H in the variable domaine V_{H}.

3. Pharmaceutical composition comprising as an active principle a mutant Fab fragment according to any of claims 1 or 2, eventually in presence of an appropriate excipient, said mutant Fab fragment being used at a concentration comprised between 0.01 mg/kg and 2 mg/kg, preferably between 0.1 and 0.4 mg/kg in weight of the patient to be treated.

4. Use of a pharmaceutical composition according to claim 3, for the preparation of a drug destined to the prevention or to the treatment of an autoimmune pathology.

5. Use according to claim 4, **characterized in that** the autoimmune pathology is selected among rheumatoid polyarthritis, psoriasis or lupus erythematosus.

6. Use of a pharmaceutical composition according to claim 3, for the preparation of a drug destined to the prevention or to the treatment of the immunologic intolerance reactions.

7. Use according to claim 6, **characterized in that** the immunologic intolerance reactions is induced following a transplantation.

8. Use of a pharmaceutical composition according to claim 3, for the preparation of a drug destined to the prevention or to the treatment of the host-versus graft reaction type.

9. Use of a pharmaceutical composition according to claim 3, for the preparation of a drug destined to the prevention or to the treatment of cancers involving the CD4 molecule.

10. Use according to claim 9, **characterized in that** the cancer involving the CD4 molecule is selected among a CD4+ lymphoma or a Cesari lymphoma.

11. Use of a pharmaceutical composition according to claim 3, for the preparation of a drug destined to the prevention or to the treatment of immunodeficiencies linked to a viral infection.

## Patentansprüche

1. Mutierendes Fragment Fab des Antikörpers Anti-CD4 13B8.2, **dadurch gekennzeichnet, dass** es das Molekül CD4 bindet und dass es eine Mutation von mindestens einem Rückstand F32, W36, C92, Y102 im variablen Bereich VH der schweren Kette und/oder C88 im variablen Bereich Vk der leichten Kette.

2. Mutierendes Fragment Fab nach Anspruch 1, **dadurch gekennzeichnet, dass** es eine Mutation C88L im variablen Bereich VK oder eine Mutation F32H, W36H, C92H oder Y102H im variablen Bereich VH.

3. Pharmazeutische Komposition, die als Aktivprinzip ein mutierendes Fragment Fab nach einem der vorstehenden Ansprüche 1 oder 2, eventuell in Anwesenheit einer geeigneten Grundmasse, wobei das besagte mutierende Fragment Fab in einer Konzentration zwischen 0,01 mg/kg und 2 mg/kg verwendet wird, bevorzugt zwischen 0,1 und 0,4 mg/kg nach Gewicht des zu behandelnden Patienten.

4. Verwendung einer pharmazeutischen Zusammensetzung nach Anspruch 3 für die Zubereitung eines Medikaments, das für die Vorbeuge oder die Behandlung einer autoimmunen Pathologie bestimmt ist.

5. Verwendung nach Anspruch 4, **dadurch gekennzeichnet, dass** die autoimmune Pathologie unter der rheumatischen Polyarthritis, dem Psoriasis oder dem Lupus Erythematodes gewählt wird.

6. Verwendung einer pharmazeutischen Zusammensetzung nach Anspruch 3 für die Zubereitung eines Medikaments, das für die Vorbeuge oder die Behandlung von Reaktionen immunologischer Unverträglichkeit bestimmt ist.

7. Verwendung nach Anspruch 6, **dadurch gekennzeichnet, dass** die Reaktion immunologischer Unverträglichkeit nach einer Transplantation induziert wird.

8. Verwendung einer pharmazeutischen Zusammensetzung nach Anspruch 3 für die Zubereitung eines Medikaments, das für die Vorbeuge oder die Behandlung von Reaktionen des Typs Transplantat dem Wirt gegenüber bestimmt ist.

9. Verwendung einer pharmazeutischen Zusammensetzung nach Anspruch 3 für die Zubereitung eines Medikaments, das für die Vorbeuge oder die Behandlung von Krebserkrankungen bestimmt ist, die das Molekül CD4 implizieren.

10. Verwendung nach Anspruch 9, **dadurch gekennzeichnet, dass** der Krebs, der das Molekül CD4 impliziert, unter einem Lymphom CD4+ oder einem Cesari Lymphom gewählt wird.

11. Verwendung einer pharmazeutischen Zusammensetzung nach Anspruch 3 für die Zubereitung eines Medikaments, das für die Vorbeuge oder die Behandlung von Immunmängeln in Verbindung mit einer viralen Infektion bestimmt ist.
